# EUROPEAN PATENT SPECIFICATION

(11) **EP 3 277 271 B1**
(45) Date of publication and mention of the grant of the patent: **21.07.2021**
(21) Application number: 16717212.1
(22) Date of filing: 31.03.2016
(51) Int. Cl.: A61K 31/192, A61K 31/352, A61K 31/37, A61P 31/12

(54) **ANTIVIRAL ACTIVITY FROM MEDICINAL MUSHROOMS AND THEIR ACTIVE CONSTITUENTS**
ANTIVIRALE AKTIVITÄT MEDIZINISCHER PILZE UND IHRE AKTIVEN INHALTSSTOFFE
ACTIVITÉ ANTIVIRALE DE CHAMPIGNONS MÉDICINAUX ET LEUR INGRÉDIENTS ACTIFS

(30) Priority: 31.03.2015 US 201562140459 P; 14.09.2015 US 201514853932
(43) Date of publication of application: 07.02.2018
(73) Proprietor: Turtle Bear Holdings, LLC, Shelton, WA 98584 (US)
(72) Inventor: STAMETS, Paul, Edward, Shelton, WA 98584 (US)
(74) Representative: Potter Clarkson
(86) International application number: PCT/US2016/025309
(87) International publication number: WO 2016/161138

(56) References cited:
- WO-A1-2015/138361
- WO-A2-02/19965
- WO-A2-02/19965
- DE-A1- 19 720 767
- DE-A1- 19 720 767
- JP-A- H02 101 013
- JP-A- H02 101 013
- US-A1- 2005 238 655
- US-A1- 2005 238 655
- US-A1- 2009 130 138
- US-A1- 2009 137 661
- US-A1- 2009 137 661
- US-A1- 2010 178 364
- US-A1- 2010 178 364
- US-A1- 2016 000 754
- CHIA-HUI LIN ET AL: "Potent Inhibitor Design Against H1N1 Swine Influenza: Structure-based and Molecular Dynamics Analysis for M2 Inhibitors from Traditional Chinese Medicine Database", JOURNAL OF BIOMOLECULAR STRUCTURE & DYNAMICS, vol. 28, no. 4, 1 February 2011 (2011-02-01), pages 471-482, XP055571341, US ISSN: 0739-1102, DOI: 10.1080/07391102.2011.10508589
- SU -YUN LYU ET AL: "Antiherpetic activities of flavonoids against herpes simplex virus type 1 (HSV-1) and type 2 (HSV-2) in vitro", ARCHIVES OF PHARMACAL RESEARCH., vol. 28, no. 11, 1 November 2005 (2005-11-01), pages 1293-1301, XP055277309, KR ISSN: 0253-6269, DOI: 10.1007/BF02978215
- LIU A L ET AL: "Structure-activity relationship of flavonoids as influenza virus neuraminidase inhibitors and their in vitro anti-viral activities", BIOORGANIC & MEDICINAL CHEMISTRY, PERGAMON, GB, vol. 16, no. 15, 1 August 2008 (2008-08-01), pages 7141-7147, XP027209760, ISSN: 0968-0896 [retrieved on 2008-06-28]
- TEJ N. KAUL ET AL: "Antiviral effect of flavonoids on human viruses", JOURNAL OF MEDICAL VIROLOGY, vol. 15, no. 1, 1 January 1985 (1985-01-01), pages 71-79, XP055570767, US ISSN: 0146-6615, DOI: 10.1002/jmv.1890150110
- RONIK KHACHATOORIAN ET AL: "Divergent antiviral effects of bioflavonoids on the hepatitis C virus life cycle", VIROLOGY, vol. 433, no. 2, 1 November 2012 (2012-11-01), pages 346-355, XP055069795, ISSN: 0042-6822, DOI: 10.1016/j.virol.2012.08.029
- PAUL SCHNITZLER ET AL: "Antiviral Activity and Mode of Action of Propolis Extracts and Selected Compounds", PHYTOTHERAPY RESEARCH., vol. 24, no. S1, 27 May 2009 (2009-05-27), pages S20-S28, XP055571361, GB ISSN: 0951-418X, DOI: 10.1002/ptr.2868
- NARESH KUMAR ET AL: "Potential applications of ferulic acid from natural sources", BIOTECHNOLOGY REPORTS, vol. 4, 1 December 2014 (2014-12-01), pages 86-93, XP055570737, ISSN: 2215-017X, DOI: 10.1016/j.btre.2014.09.002
- H.D. GRAVINA ET AL: "In vitro assessment of the antiviral potential of trans-cinnamic acid, quercetin and morin against equid herpesvirus 1", RESEARCH IN VETERINARY SCIENCE., vol. 91, no. 3, 1 December 2011 (2011-12-01), pages e158-e162, XP055570771, GB ISSN: 0034-5288, DOI: 10.1016/j.rvsc.2010.11.010
- LINDEQUIST U ET AL: "THE PHARMACOLOGICAL POTENTIAL OF MUSHROOMS", EVIDENCE-BASED COMPLEMENTARY AND ALTERNATIVE MEDI, XX, XX, vol. 2, no. 3, 1 January 2005 (2005-01-01) , pages 285-299, XP009076038,
- N.A. AWADH ALI ET AL: "Antiviral activity of Inonotus hispidus", FITOTERAPIA., vol. 74, no. 5, 1 July 2003 (2003-07-01), pages 483-485, XP055277389, IT ISSN: 0367-326X, DOI: 10.1016/S0367-326X(03)00119-9
- JIANMIN WANG ET AL: "Anti-Enterovirus 71 Effects of Chrysin and Its Phosphate Ester", PLOS ONE, vol. 9, no. 3, 5 March 2014 (2014-03-05), page e89668, XP055277290, DOI: 10.1371/journal.pone.0089668
- M.J Alves ET AL: "Antimicrobial activity of phenolic compounds identified in wild mushrooms, SAR analysis and docking studies", Journal of Applied Microbiology, 1 August 2013 (2013-08-01), pages 346-357, XP055277451, England DOI: 10.1111/jam.12196 Retrieved from the Internet: URL:http://onlinelibrary.wiley.com/doi/10. 1111/jam.12196/epdf
- CHANG HWA HWANG ET AL: "Chlorinated Coumarins from the Polypore Mushroom Fomitopsis officinalis and Their Activity against Mycobacterium tuberculosis", JOURNAL OF NATURAL PRODUCTS., vol. 76, no. 10, 25 October 2013 (2013-10-25), pages 1916-1922, XP055277065, US ISSN: 0163-3864, DOI: 10.1021/np400497f cited in the application
- CHIA-HUI LIN ET AL: "Potent Inhibitor Design Against H1N1 Swine Influenza: Structure-based and Molecular Dynamics Analysis for M2 Inhibitors from Traditional Chinese Medicine Database", JOURNAL OF BIOMOLECULAR STRUCTURE & DYNAMICS, vol. 28, no. 4, 1 February 2011 (2011-02-01), pages 471-482, XP055571341, US ISSN: 0739-1102, DOI: 10.1080/07391102.2011.10508589
- SU -YUN LYU ET AL: "Antiherpetic activities of flavonoids against herpes simplex virus type 1 (HSV-1) and type 2 (HSV-2) in vitro", ARCHIVES OF PHARMACAL RESEARCH., vol. 28, no. 11, 1 November 2005 (2005-11-01), pages 1293-1301, XP055277309, KR ISSN: 0253-6269, DOI: 10.1007/BF02978215
- LIU A L ET AL: "Structure-activity relationship of flavonoids as influenza virus neuraminidase inhibitors and their in vitro anti-viral activities", BIOORGANIC & MEDICINAL CHEMISTRY, PERGAMON, GB, vol. 16, no. 15, 1 August 2008 (2008-08-01), pages 7141-7147, XP027209760, ISSN: 0968-0896 [retrieved on 2008-06-28]
- TEJ N. KAUL ET AL: "Antiviral effect of flavonoids on human viruses", JOURNAL OF MEDICAL VIROLOGY, vol. 15, no. 1, 1 January 1985 (1985-01-01), pages 71-79, XP055570767, US ISSN: 0146-6615, DOI: 10.1002/jmv.1890150110
- RONIK KHACHATOORIAN ET AL: "Divergent antiviral effects of bioflavonoids on the hepatitis C virus life cycle", VIROLOGY, vol. 433, no. 2, 1 November 2012 (2012-11-01), pages 346-355, XP055069795, ISSN: 0042-6822, DOI: 10.1016/j.virol.2012.08.029
- PAUL SCHNITZLER ET AL: "Antiviral Activity and Mode of Action of Propolis Extracts and Selected Compounds", PHYTOTHERAPY RESEARCH., vol. 24, no. S1, 27 May 2009 (2009-05-27), pages S20-S28, XP055571361, GB ISSN: 0951-418X, DOI: 10.1002/ptr.2868
- NARESH KUMAR ET AL: "Potential applications of ferulic acid from natural sources", BIOTECHNOLOGY REPORTS, vol. 4, 1 December 2014 (2014-12-01), pages 86-93, XP055570737, ISSN: 2215-017X, DOI: 10.1016/j.btre.2014.09.002
- H.D. GRAVINA ET AL: "In vitro assessment of the antiviral potential of trans-cinnamic acid, quercetin and morin against equid herpesvirus 1", RESEARCH IN VETERINARY SCIENCE., vol. 91, no. 3, 1 December 2011 (2011-12-01), pages e158-e162, XP055570771, GB ISSN: 0034-5288, DOI: 10.1016/j.rvsc.2010.11.010
- LINDEQUIST U ET AL: "THE PHARMACOLOGICAL POTENTIAL OF MUSHROOMS", EVIDENCE-BASED COMPLEMENTARY AND ALTERNATIVE MEDI, XX, XX, vol. 2, no. 3, 1 January 2005 (2005-01-01) , pages 285-299, XP009076038,
- OU S ET AL: "FERULIC ACID: PHARMACEUTICAL FUNCTIONS, PREPARATION AND APPLICATIONS IN FOODS", JOURNAL OF THE SCIENCE OF FOOD AND AGRICULTURE, WILEY & SONS, CHICHESTER, GB, vol. 84, no. 11, 30 August 2004 (2004-08-30), pages 1261-1269, XP001205991, ISSN: 0022-5142, DOI: 10.1002/JSFA.1873

## Description

### TECHNICAL FIELD

The present invention relates to antiviral compositions as defined in Claim 1 based upon constituents isolated from or contained within medicinal mushroom mycelia, or the corresponding synthetic molecules, that are shown to be useful in reducing pathogenic viruses, and treating viral infections; in particular viruses that afflict animals, including, but not limited to, humans, bees, pigs, bats, and birds, resulting in a reduction of disease causing viruses, their pathogenicity and/or infectivity in both the animal host and the environment.

### BACKGROUND

Medicinal mushrooms have been used for thousands of years for a wide assortment of ailments. Traditionally the mushroom fruitbody has been used. Scientists have extensively studied extracts of the fruitbodies over the past decades. Although numerous papers have been published showing hot water extracts of mushrooms and their mycelia can activate immune systems and can be anti-inflammatory, comparatively few have elucidated the benefits of the alcohol fractions. The current invention describes novel contributions to the field of medicinal mushroom research, particularly discoveries pertaining to antiviral activity of alcohol extracted mushroom mycelium and the active constituents contained within them.

Scientists are now discovering that viral infection challenges and degrades the immune system in multiple ways including inflammation, which can lead to cellular damage from free radicals, a cofactor in carcinogenesis, and to cancers caused by oncoviruses. Worldwide, the World Health Organization (WHO) International Agency for Research on Cancer estimated that in 2002 17.8% of human cancers were caused by infection, with 11.9% being caused by 1 of 7 different viruses. The 7 viruses that are known to cause cancer include three herpes oncoviruses: Epstein-Barr aka human herpesvirus 4 (HHV-4); human herpesvirus 6 (HHV-6) and human herpesvirus 8 (HHV-8). HHV-6 is implicated in the development of lymphomas, leukemia, cervical cancers, Karposi sarcoma, and brain tumors. Other oncoviruses include the polyoma virus that causes Merkel cell carcinoma (MCC), the human papillomaviruses (HPV 16 and 18) which cause cervical cancer, anal cancer, oropharyngeal cancers, vaginal cancers, vulvar cancers and penile cancers; hepatitis B and C, which cause liver cancer; and the human T-lymphotropic viruses (HTLV), which cause T-cell leukemia and T-cell lymphoma. Four HTLVs are well known. HTLV-1 and HTLV-2 are involved in epidemics, infecting 15-20 million people worldwide. In the United States, hepatitis C infection is estimated at 2.7 million and 700,000-1.4 million persons are estimated to be infected with hepatitis B. HTLVs can be more prevalent in some geographical regions than others, infecting around 1% of Japan's population. Rates among volunteer blood donors in the U.S. average 0.016% but in parts of Africa, reports of 15% have been recorded.¹

As science progresses, more oncoviruses and virally-mediated oncogenic pathways are likely to be discovered. Since immunity is based on many complex factors, pathogenic viruses which have not been known to specifically cause cancer may contribute to carcinogenesis by causing inflammation, free radicals, and reducing the number of immune cells that would otherwise keep cancer and co-infections at bay. Nature is a number's game, and the balance between health and disease is imperiled by infections. When immunity is lowered, the human body is less able to eradicate cancer cells, which would otherwise be kept in check, and deleterious inflammatory pathways further challenge health. Thus mortality rates rise with compounded infections. Hence there is a need for compositions that reduce oncoviruses and also reduce viruses that cause inflammation and immune deactivation, contributory to oncogenesis.

Viral epidemics and pandemics represent increasing threats to global health as zoonotic diseases spread, jumping host species, recombining, and potentially mutating into more virulent forms. The need for additional anti-influenza drugs is important in maintaining active countermeasures against pandemics. As an example, when H1N1 flu virus swept the world in 2010, the two most popularly effective antivirals were Tamiflu® (Oseltamivir) and Relenza® (Zanamivir); which were useful, at best, for shortening the disease period by approximately a day. In less than one year, the novel H1N1 virus evolved to increasingly resist Tamiflu® applications, and the drug has largely become ineffective against downstream populations of the heritage H1N1 swine flu viruses. Although some antiviral medicines may be effective at present, it is vital that researchers investigate diverse therapeutic agents in order to combat viral outbreaks from rapidly evolving strains. The ease and speed with which numerous varieties of flu virus mutate to become drug resistant is particularly concerning.

In the spring of 2015, wild birds from Asia carried H5N8 viruses to North America, which co-mingled with bird flu variants and mutated into a highly pathogenic H5N2 virus. This virus resulted in the killing - both from the virus and euthanasia - of tens of millions of birds and threatened the multibillion dollar chicken and turkey industry. The H5N2 virus has mutated into H5N1 variants, and given the number of hosts in wild and domesticated birds, continued mutations could evolve a strain of the flu that could leap to humans, causing a pandemic and severe devastation to our global economies, our food biosecurity and human health. Given that flu viruses can be spread via airborne, direct and secondary contacts (via vehicles, shoes, clothing, washcloths, dollar bills, flies, mites, etc.) and that flu viruses can survive in mucous droplets for up to 17 days, the threat of a flu pandemic spreading to humans greatly concerns specialists in virology, public health and defense. Finding methods and compositions to reduce the viral pathogen payloads vectored by host animals and fomites will greatly serve the public interest. Moreover, since most vaccines have limited (but focused) utility against only a few flu variants, finding broad based solutions to preventing and reducing the threat from multiple flu viruses in particular, and diverse viruses in general, is of paramount importance.

Medicinal mushrooms have been ingested as food and as therapy for hundreds, and in some cases, thousands of years. This is strong support for their safe ingestion, making them appealing candidates in the search for new antiviral agents. In addition, the compounds disclosed herein may be resident ingredients within well-known foods, which, when isolated and concentrated can function as drugs. The difference here then between a food and a drug is that a drug is typically an isolated molecule presented in a form at a high purity (i.e. >90%), and used at a high dose in treating a disease. One of the first mushrooms recognized for its antiviral activity was *Fomes fomentarius,* a hoof-shaped wood conk that was found to inhibit the tobacco mosaic virus.² More recently, derivatives of the Gypsy mushroom, *Rozites caperata,* were found by Piraino *et al.* to significantly inhibit the replication and spread of *Varicella zoster* (the 'shingles' virus), influenza A and B, and herpes simplex I and II. Sarkar *et al.* have also identified activity against herpes simplex I in an extract of Shiitake mushrooms (*Lentinula edodes*).^{3,4} Collins and Ng have identified a polysaccharopeptide inhibiting HIV type 1 infection from Turkey Tail mushrooms (*Coriolus versicolor* = *Trametes versicolor*).⁵ Brandt and Piraino, and Stamets have also published summaries of the antiviral properties of some mushrooms species.^{6,7}

The prevailing adamant belief by those skilled in the science of medicinal mushroom research is that the only benefits from medicinal mushroom extracts must come from hot water extraction. As three noted experts, skilled in the art, and authors of scientific papers and books on the medicinal properties of mushrooms, have published: "Hot water extracts are the only form of mushroom preparation ever used in Traditional Chinese Medicine (TCM), and the only form of mushroom supplement ever used, tested or studied in the scientific and medical research." (The Health Benefits of Medicinal Mushrooms, 2005, Dr. Mark Stengler).⁸

According to John Seleen of Mushroom Science (currently on his Mushroom Science website), "Few people realize how much research has been conducted on medicinal mushrooms; more than 2,000 studies have been published in just the last 10 years, and all of these studies have used hot water extracts. In fact, hot water extracts are the only type of medicinal mushroom preparation that has actual proof of effectiveness for supporting immune health. It is not often that you have absolute consensus between 1,000's of years of herbal practice and every scientific study ever published on that same subject, but that is the case with medicinal mushrooms. All sources and traditions agree, medicinal mushrooms must be extracted with hot water when used for immune support, and hot water extracts are the only type of mushroom supplement validated by the research." (September 10, 2015).

Additionally, a 2015 'white paper' by Jeffery S. Chilton, *Redefining Medicinal Mushrooms: A new scientific screening program for active compounds* states that if mycelium is grown on rice, and not wood, that "Without the natural precursors, basidiomycete mycelium in sterile culture produces few of the important secondary metabolites." Thus these three experts, skilled in the art, and greatly influential, are unanimous in discrediting any significant activity of non-hot water mycelial extracts, especially when mycelium is grown on grains such as rice. Thus they teach away from the specifics of this invention.

With the advent of tissue culture of mycelium in the early part of the 20^{th} century, this new mushroom life stage (the mycelium as opposed to the mushroom fruit bodies) became available for testing bioactivity. This newly available fungal form opened up new frontiers for natural product research. However, pharmaceutical companies studying mushroom-based natural products, typically and more inexpensively, analyze the fruitbodies, and in doing so miss the antiviral activities this inventor has discovered that are expressed during the mycelium life stage.

From a practical point of view, pharmaceutical researchers find it easier to collect and analyze a mushroom rather than to laboriously culture it and then analyze the mycelium. This standard approach has a reasonable rationale: many species of mushroom forming fungi do not grow, or are too slow to grow in *in vitro* culture compared to other fungi such as molds. Additionally, the mushroom fruitbodies are made of compacted mycelium - dense with tissue - and hence would seemingly be a better resource for bioprospecting than the more loosely netted mycelium. This would explain why there is little prior art on the mycelium of mushroom species being anti-virally active. Typically, when a pharmaceutical company screens mushroom-based natural products, they analyze large sets of species. If they do not find activity in the natural form (the mushroom fruitbody or carpophore), they move on to other species without further exploring a negative result, based upon the mistaken belief that the activities of the mushrooms would be the same as the mycelium and that all strains or cultivars of a species would possess the same antiviral activity. This is understandable since the prevailing belief is that the mushroom is simply composed of compacted mycelium and the two would share, in common, the same constituents.

Recent genomic research shows that more genes are turned on during the mycelial stage of development than in the reproductive structure of the mushroom fruitbodies. As was noted by Li *et al.,* 2013, "The protein-coding genes were expressed higher in mycelia or primordial stages compared with those in the fruiting bodies.^{"9} The inventor's practices have inadvertently laid claim to or supported this statement without prior knowledge that more genes are up-regulated during mycelial growth than fruitbody (mushroom) formation. This was not known, nor obvious, at the time when this patent applicant filed his first provisional antiviral patent application U.S. 60/534,776 on Jan. 6, 2004.

Remarkably, and unexpectedly, the author's discovery that the alcohol soluble extracts of *Ganoderma lucidum* (*Ganoderma lucidum* var. *resinaceum*) mycelium showed anti-flu activity is novel in that it is in direct contradiction to past results that alcohol extracts from fruitbody extracts had no activity. This is unique in that it is counter-intuitive as conventional thinking would lead most scientists to believe that the activity of both forms would share commonality of effects.

Seong-Kug Eo tested both water soluble and alcohol soluble fractions from the fruitbodies of *Ganoderma lucidum.¹⁰* The methanol soluble compounds were labeled as "GLMe," for *"Ganoderma lucidum* methanol fraction" and "GLhw" for "G. *lucidum* hot water."- Their conclusions showed that the methanol (alcohol) soluble fractions had no activity against flu viruses: "The carpophores of G. *lucidum* (500 g) were disrupted and extracted with hot water for 8 h. The water extract was concentrated to a 10th of the original volume, and three volumes of ice cold EtOH added to precipitate the high molecular weight components. After standing out overnight at 4°C, it was centrifuged and the precipitates were lyophilized, and GLhw (3.30 g) as a brownish substance was obtained. Eight methanol soluble substances (GLMe) were isolated by organic solvents on the basis of differences in the net electric charge. GLMe-1,-2, -4 and -7 isolated from the MeOH fraction exhibited inhibitory effects, especially on the cytopathic effects induced by VSV Indiana and New Jersey strains at concentrations which did not show cytotoxicity against Vero cells; however, they exhibited no effect on the other viruses such as HSV and influenza A virus."

The author notes that this article has been referenced, as of this date, August 18, 2015, 2,090 times according to Google and 3,570 times by Bing search engines, showing that Seong-Kug Eo *et al.*'s (1999) statement that the alcohol soluble fractions of *Ganoderma lucidum* were inactive against flu and herpes viruses was well established in the scientific literature.

This discovery by Seong-Kug Eo *et al.* (1999) teaches away from Stamets Patent No. 8,765,138 (2014), the latter of which discloses that alcohol extracts of the *Ganoderma lucidum* mycelium were highly active against flu and herpes viruses. Conventional wisdom has been that the fruitbodies (mushrooms) of *Ganoderma lucidum* held the most diverse bioactive constituents and that the mycelium is less active. Moreover, since the fruitbodies are composed of mycelium, that there would be differences between extracts made from mushrooms vs mycelia would have been seen, by those currently and historically skilled in preparations of mushrooms, to be a factual contradiction. Hence, the inventor's results were both novel and nonobvious at the time of this inventor's first antiviral patent applications wherein he found that the EtOH/H₂O extracts of fruitbodies of Agarikon (*Fomitopsis officinalis*) were inactive against pox, flu and herpes viruses whilst the EtOH/H₂O extracts from the living mycelium of this species were active against the same viruses.

US2010/178364 describes methods for preventing or attenuating microcompetition between a foreign polynucleotide and a cellular polynucleotide or attenuating an effect of such microcompetition.
US2009/137661 describes compositions for reducing the activity of a cervical cancer cell. The composition includes at least one of the following compounds: isopsoralen, triptolide, baicalein, gallic acid, quercetin, gossypol-acetic acid, baicalin, berberine hydrochloride, and derivatives thereof in a sufficient amount to reduce the activity of the cervical cancer cell in the subject.
WO02/19965 describes a method of inhibiting heat shock protein-dependent virus replication in cells and in animals. Also disclosed is a method of identifying compounds which inhibit heat shock protein-dependent virus replication.

### SUMMARY

The present invention provides a composition for use in treating a pathogenic virus infection comprising one or more of syringic acid, trans-cinnamic acid, trans-ferulic acid, salts thereof, esters thereof, or combinations thereof, wherein the pathogenic virus is human Papillomavirus (HPV). In one aspect, syringic acid, trans-cinnamic acid and trans-ferulic acid have an antiviral effect Selectivity Index 50 (SI₅₀)≥10 against the human papillomavirus (HPV), and trans-cinnamic acid and trans-ferulic acid have an antiviral effect Selectivity Index 50 (SI₅₀) ≥100 against the human papillomavirus (HPV). In another aspect, the antiviral effect has a selectivity index (SI₅₀= CC₅₀/EC₅₀) against the pathogenic virus SI₅₀≥5. In another aspect, the antiviral effect has a selectivity index (SI₅₀= CC₅₀/EC₅₀) against the pathogenic virus SI₅₀≥10. In another aspect, the antiviral effect has a selectivity index (SI₅₀= CC₅₀/EC₅₀) against the pathogenic virus SI₅₀≥100. In another aspect, the composition additionally has an antibacterial effect. As described herein, the composition may additionally comprise an extract of mycelium of a fungus. In another aspect described herein, the fungus comprises *Polyporales, Hymenochaetales,* or *Agaricales.The* fungus comprises *Antrodia, Fomitopsis, Ganoderma, Hypsizygus, Inonotus, Piptoporus, Pleurotus, Trametes,* or *Polyporus* species. In another aspect described herein, the fungus comprises *Antrodia camphoratus, Antrodia cinnamomea, Fomitopsis officinalis, Ganoderma annulare, Ganoderma applanatum, Ganoderma brownii, Ganoderma lucidum, Ganoderma lingzhi, Ganoderma resinaceum, Inonotus obliquus, Trametes versicolor, Hypsizygus tessulatus, Hypsizygus ulmarius,* or *Pleurotus ostreatus.*

The composition for use may be administered as a daily dose of a composition for a period of time from 10 to 60 days to a patient suffering from the pathogenic virus infection, wherein the daily dose comprises about 0.001 to 2 grams of the composition, wherein the composition is as defined in Claim 1. In one aspect, the daily dose comprises about 0.1 to 1.5 grams of the composition. In another aspect, the daily dose comprises about 0.25 to 1.4 grams of the composition. In another aspect, the daily dose comprises about 1,000 milligrams of the composition.

Also described herein is a method for manufacturing a dosing form for treating, ameliorating, mitigating, alleviating, reducing and curing a virus infection comprising: formulating a composition into a dosage form comprising sprays, capsules, tablets, elixirs, emulsions, lozenges, suspensions, syrups, pills, lotions, epidermal patches, suppositories, inhalers, or injectables, wherein the composition comprises one or more of ethyl 7-chloro-2-oxo-4-phenyl-2H-chromen-3-carboxylate, vanillic acid, chrysin, quercetin hydrate, rutin hydrate, syringic acid, *trans*-cinnamic acid, *trans*-ferulic acid, salts thereof, esters thereof, or combinations thereof, and wherein the composition has an antiviral effect against a pathogenic virus comprising one or more of herpes *Varicella zoster* virus, Epstein-Barr virus, herpes simplex I and II viruses, human papillomaviruses (HPV), poliovirus, or norovirus.

Also described herein is a pharmaceutical composition comprising one or more extracts from one or more fungi mycelia. The composition additionally comprises an active compound. The active compound comprises one or more extracted, isolated, synthetic or manufactured active compounds. The fungi comprise one or more of *Antrodia, Fomitopsis, Ganoderma, Hypsizygus, Inonotus, Piptoporus, Pleurotus, Trametes* and *Polyporus* species or the active compound comprises one or more of ethyl 7-chloro-2-oxo-4-phenyl-2H-chromen-3-carboxylate, vanillic acid, chrysin, quercetin hydrate, rutin hydrate, syringic acid, trans-cinnamic acid, trans-ferulic acid, dehydrosulphurenic acid, eburicoic acid, salts thereof, esters thereof, or combinations thereof. The pharmaceutical composition described herein has antiviral activity against herpes viruses, herpes *Varicella zoster* virus, Epstein-Barr virus, herpes simplex I and II viruses, human Papillomaviruses (HPV), influenza viruses, hepatitis viruses, poliovirus, or norovirus.

Patients often co-harbor multiple viruses, and as immunity declines, multiple viruses can proliferate. The combination of multiple viruses can exacerbate pre-existing conditions, cause new diseases to be expressed, and confounds medical protocols due to the complexity of factors that are presented. As such, having a novel combination of active antiviral molecules is useful and unique. Combining these molecules with extracts of medicinal mushroom mycelia adds additional benefits beyond simple antiviral activity. These additional benefits include but are not limited to reducing inflammation, activating P21 and P53 tumor regression pathways, enhancing Natural Killer Cell, Macrophage and cytotoxic T cell readiness, activity, populations and ability to target diseased cells, bolstering overall host defense readiness of patients.

The present invention also provides a dosing form for treating, ameliorating, mitigating, alleviating, reducing and curing a virus infection comprising: formulating a composition into a dosage form comprising sprays, capsules, tablets, elixirs, emulsions, lozenges, suspensions, syrups, pills, lotions, epidermal patches, suppositories, inhalers, or injectable, wherein the composition is as defined in Claim 1 In another aspect, the composition additionally comprises any active medicinal compound. In another aspect, the active compound comprises one or more extracted, isolated, synthetic or manufactured active compounds.

Alsodescribed herein is a method for preparing a pharmaceutical composition comprising one or more extracts from one or more fungi mycelia, the means comprising cultivating a pure mushroom mycelium on a solid substrate or liquid substrate under appropriate light and extracting the mycelium with one or more solvents, water, cold water, hot water, water/ethanol or a solvent, and one or more steps of combining alcohol extracts and water extracts in ratios ranging from 1:1 to 1:99, adjusting water/ethanol extracts alcohol content to one or more of 22-23% ethanol by volume or 22-50% ethanol by volume or 25-35% ethanol by volume or 30-40% ethanol by volume or 35-45% ethanol by volume, filtering, partially adding ethanol to precipitate water-soluble polysaccharides, precipitating solids or a precipitate, separating a supernatant from solids or a precipitate, concentrating a supernatant, partially or fully removing a solvent or liquid, drying, isolating or purifying an active ingredient, isolating or purifying a fraction, isolating or purifying an active compound, or combining with CBD (cannabidiol), providing physical or chemical method or means for increasing bioavailability or formulating extracts or active principal ingredients into lotions or other methods or means of delivery to a patient.

Another embodiment a pharmaceutical composition produced by any of the means described herein. In one aspect, the composition comprises one or more of syringic acid, rans-cinnamic acid, trans-ferulic acid, salts thereof, esters thereof, or combinations thereof.

Also described herein is a method for preparing a composition comprising one or more of ethyl 7-chloro-2-oxo-4-phenyl-2H-chromen-3-carboxylate, vanillic acid, chrysin, quercetin hydrate, rutin hydrate, syringic acid, trans-cinnamic acid, trans-ferulic acid, salts thereof or esters thereof molecules, comprising isolating, purifying, synthesizing or manufacturing the molecules and combining in various ratios from 1:1 to 99:1 by weight percentage, including all integers within the specified ratio range, or combining any FDA approved ingredients.

Also described herein is a means for preparing a composition comprising one or more of ethyl 7-chloro-2-oxo-4-phenyl-2H-chromen-3-carboxylate, vanillic acid, chrysin, quercetin hydrate, rutin hydrate, syringic acid, trans-cinnamic acid, trans-ferulic acid, salts thereof, esters thereof, or combinations thereof comprising isolating, purifying, synthesizing or manufacturing the molecules and combining in various ratios from 1:1 to 99:1 by weight percentage, including all integers within the specified ratio range, or combining any FDA approved ingredients.

Another embodiment is a pharmaceutical composition produced by any of the means described herein. In one aspect, the composition is as defined in Claim 1.

### DISCLOSURE OF INVENTION

"Extract" as used herein may refer to a crude extract of mycelium, a partially purified extract or an active compound, ingredient or molecule isolated from such extract. Any means for extraction known to the art may be utilized.

In view of the lack of use of medicinal mushroom mycelium and its inherent components to protect against viruses afflicting animals, the present invention provides novel compositions and methods of using such agents or their cheaper synthetic equivalents.

The present invention offers a unique approach to viral control by utilizing molecular constituents, more specifically discrete active pharmacological ingredients, resident within aqueous-ethanol extracts from the living mycelium of *Antrodia, Fomes, Fomitopsis, Ganoderma, Inonotus, Schizophyllum, Phellinus, Piptoporus, Trametes* and other taxa in the Polyporaceae, and a range of active principle ingredients coded by gene sequences from the mycelia of related medicinal mushrooms to combat harmful viruses.

This patent focuses on EtOH/H₂O (ethyl alcohol/water) extracts from living mycelium to discover new medicines, particularly antivirals. Methanol, and other solvents known to the art can be used but the inventor focused on ethanol because ethanol can be consumed whereas methanol is highly toxic (causing blindness) if consumed. New antiviral molecules are disclosed that are components within the extracts of living mycelium.

Hence this invention not only provides the first evidence that extracts and molecules within these extracts reduce some oncoviruses and also reduce viruses that cause inflammation and immune deactivation, contributory to oncogenesis. Not only do these extracts and active constituents reduce the pathogenicity of viruses but reduce also, as a consequence, cancer risk. Therefore, the active agents found by this inventor can be combined for anticancer compositions, significantly enhancing the benefits of other anticancer drugs and integrative medicine programs for increasing the quality of life of cancer patients.

This invention offers a broad bioshield of defense from pathogenic viruses for humans, birds, bees, pigs, and other animals using extracts from medicinal mushrooms and their active pharmaceutical ingredients, active molecules or active compounds. These extracts contain many antiviral compounds, the use of which is new to science, unanticipated and nonobvious.

The inventor surveyed the scientific literature up to 2001 and wrote a literature review for the peer-reviewed journal *HerbalGram.*¹¹ The antivirals from mushroom species - up to that date - were made from fruitbody extracts, except in the case of Shiitake (LEM from *Lentinula edodes*) and Turkey Tail (PSP and PSK from *Trametes versicolor*). LEM, PSP and PSK are isolated using hot water as the primary, "first step" extracting solvent. Noteworthy is that LEM, PSK and PSP precipitate out of solution when EtOH levels exceed 25-35% - a method this inventor practices. Using the clarified supernatant, devoid of the precipitant rich LEM, PSK and PSP compounds, this inventor opposed conventional thinking. Removal of ethanol insoluble beta-glucans and proteins, helped, in this inventor's opinion, unmask a new set of antiviral agents. However, the inventor, in 2001, reflecting the current conventional wisdom of the science stated in the inventor's peer reviewed botanical journal article¹² that the only antivirals known are from hot water extraction and these are polysaccharide or polysaccharide protein rich (beta-glucan) based antivirals present both in the mycelium and in the fruiting bodies. As it turns out, this was not necessarily true, as was subsequently discovered by this inventor, much to his surprise, and contrary to conventional wisdom to those skilled in the art.

### DETAILED DESCRIPTION OF THE INVENTION

The present invention provides a composition for use in treating a pathogenic virus infection comprising one or more of syringic acid, trans-cinnamic acid, trans-ferulic acid, salts thereof, esters thereof, or combinations thereof, wherein the pathogenic virus is human Papillomavirus (HPV). The invention includes active principle ingredients found in the combination of products from the mycelia of multiple mushroom species in a form to have the accumulated effect of restricting the growth, spread and survivability of viruses in animals, especially humans, birds and bees. The present invention also includes the combination of products from multiple mushroom species in a form useful for preventing, treating, alleviating, mitigating, ameliorating or reducing viruses, including oncoviruses, in animals including humans. Such forms may have the additional advantages of functioning as antibacterials, antiprotozoals, immunomodulators, nutraceuticals and/or prebiotics as well as enhancing innate immunity defense mechanisms and host immune response, resulting in healing.

Mushrooms exhibiting medicinal properties are numerous. Some species and subspecies ("strains") vary in their proportionalities of constituents. This inventor has found a surprising array of antiviral molecules spread amongst different families of molecules. More particularly, polyphenols within the polyporales (the so called 'polypore' mushrooms) contain many of the antiviral molecules discovered by this inventor, many of which are expressed as extracellular, primary and secondary metabolites. These molecules are variously soluble in a wide range of solvents, from highly polar water to hexane and oils, which are least polar. The polyphenols, lipids and fatty acids of greatest interest for novel antiviral activity are particularly, but not exclusively, those that are soluble in non-polar solvents.

The aqueous-alcohol extracts of antiviral and antibacterial mushroom mycelia, especially in the polyporaceae, more specifically *Fomitopsis officinalis* (=*Laricifomes officinalis*), *Fomitopsis pinicola, Ganoderma annulare, Ganoderma lingzhi, Ganoderma lucidum, Ganoderma lucidum* var. *resinaceum* (=*Ganoderma resinaceum), Ganoderma applanatum, Ganoderma brownii, Ganoderma atrum, Ganoderma oregonense, Ganoderma sinense, Ganoderma tsugae,* and other *Ganoderma* species, *Inonotus obliquus, Piptoporus betulinus, Schizophyllum commune,* and *Trametes versicolor* contain molecules highly active in reducing pathogenic viruses. Additional species likely to provide a reservoir of antiviral molecules include but are not limited to Polyporales and Hymenochaetales such as *Antrodia cinnomonea, Fomitiporia robusta, Fomes fomentarius, Ganoderma curtisii, Ganoderma lingzhi, Grifola frondosa, Heterobasidion annosum, Inonotus hispidus, Inonotus andersonii, Inonotus dryadeus, Irpex lacteus, Laetiporus cincinnatus, Laetiporus sulphureus, Laetiporus conifericola, Lentinula edodes, Lenzites betulina, Phanerochaete chrysosporium, Phaeolus schweinitzii, Phellinus baumii, Phellinus igniarius, Phellinus linteus, Phellinus pini, Polyporus elegans, Phanerochaetes chrysosporium, Phaeolus schweitnitzii, Stereum complicatum, Stereum hirsutum, Stereum ostrea, Trametes elegans, Trametes gibbosa, Trametes hirsuta, Trametes villosa, Trametes cingulata, Trametes ochracea, Trametes pubescens, Trametes ectypa, Trametes aesculi, Wolfiporia cocos,* and Agaricales such as *Agaricus augustus, Agaricus blazei, Agaricus bonardii, Agaricus brasiliensis, Agaricus campestris, Agaricus lilaceps, Agaricus placomyces, Agaricus subrufescens, Agaricus sylvicola, Agrocybe pediades, Agrocybe aegerita, Agrocybe arvalis, Agrocybe praecox, Amanita muscaria, Amanita gemmata, Amanita pantherina, Amanita phalloides, Amanita virosa, Amanita pachycolea, Amanita vaginata, Clitocybe odora, Clitocybe dealbata, Clitocybe dilitata, Conocybe cyanopus, Conocybe lacteus, Conocybe rickenii, Conocybe smithii, Conocybe tenera, Coprinopsis atrementaria, Coprinopsis nivea, Coprinopsis lagopus, Coprinus comatus, Coprinus micaceus, Galerina autumnalis, Galerina marginata, Galerina venenata, Gymnopus hydrophilus, Gymnopilus peronatus, Hypholoma aurantica* (*Leratiomyces ceres*), *Hypholoma capnoides, Hypholoma fasciculare, Hypholoma sublateritium, Hypsizygus marmoreus, Hypsizygus tessulatus, Hypsizygus ulmarius, Lentinus ponderosus, Lepiota procera* (*Macrolepiota procera*)*, Lepiota rachodes* (*Chlorophyllum rachodes*), *Lepista nuda, Mycena alcalina, Mycena pura, Mycena aurantiadisca, Panellus serotinus, Panaeolus foenisecii, Panaeolus subbalteatus, Pleurotus columbinus, Pleurotus ostreatus, Pleurotus cystidiosus, Pleurotus pulmonarius, Pleurotus sapidus, Pleurotus tuberregium, Panellus stipticus, Panellus serotinus, Pluteus cervinus, Psathyrella aquatica, Psathyrella condolleana, Psathyrella hydrophila, Psilocybe allenii, Psilocybe azurescens, Psilocybe baeocystis, Psilocybe caerulescens, Psilocybe coprophila, Psilocybe cubensis, Psilocybe cyanescens, Psilocybe ovoideocystidiata, Psilocybe semilanceata, Psilocybe stuntzii, Psilocybe subaeruginosa, Psilocybe weilii, Stropharia aeruginosa, Stropharia coronilla, Stropharia coronilla, Stropharia cyanea, Stropharia rugoso-annulata, Stropharia semiglobata, Stropharia semigloboides, Stropharia squamosa, Stropharia thrausta, Stropharia umbonotescens, Termitomyces robusta, Volvaria bombycina, Volvariella volvacea,* and ascomycetes such as *Metarhizium anisopliae, Metarhizium acridum, Beauveria bassiana, Cordyceps capitata, Cordyceps militaris, Cordyceps sinensis* sensu lato, *Cordyceps subsessilis, Ophiocordyceps sinensis,* and *Ophiocordyceps unilateralis.*

The effect of the antiviral and antibacterial components within the aforementioned mushroom species and their relatives may be the decisive factor that improves survivability from infection in many animals including humans, dogs, cats, horses, cows, pigs, birds, fish, insects (including bees) and other wild and domesticated animals. Moreover, these mushroom extracts, and natural extracted or synthetic versions of the compounds contained in such extracts, can enhance and be combined with a wide range of conventional anticancer therapies, including chemotherapies using herceptin, tamoxifen, taxol, interferon alpha, as well as vaccines, gene therapies, including incorporating nanobots, radiological, immunological, sonic, photonic, electrical, cold shock, electromagnetic, and microbiomic and other cancer therapies.

Lowering the cancer-causing effects of viruses and bacteria are a consequence of and are derived from the antiviral, antibacterial and other medicinal effects of polypore mushroom mycelia in particular, and the mycelia from other medicinal mushrooms in general, as described in U.S. Patent No. 8,765,138, issued 07/01/2014 by the inventor. By employing extracts and the derivative active ingredients from these antiviral and antibacterial fungi, improvement in survivability of patients, including both humans and animals, can be significantly realized.

### Methods and Means of Extraction

This inventor has discovered that cold water or ambient temperature (75 °F ≈ 24 °C) EtOH/H₂O (ethanol/water) extractions as outlined in U.S. Patent No. 8,765,138 (2014) and U.S. Patent Application No. 14/641,432 (2015), are effective for extracting high potency antiviral molecules and are immune supporting, contrary to prevailing opinions of experts skilled in the art who argue that only hot water extracts are useful for extracting medicinal mushroom-based immune supporting compounds.

In contrast, the inventor discovered that cold extraction yielded antiviral actives contrary to conventional thinking, which advocates hot extraction (see paragraphs 0009-0011 above). "Cold" is an empirical perception. The inventor defines "cold" as being < 98.6 °F (<37 °C) the average temperature of a human and "hot" as being >98.6 °F (>37 °C). The inventor used cold extraction methods, more specifically at room temperature (≈ 72-75 °F; ≈ 22-24 °C), to make ethanol/water extracts of the mycelium of *Fomitopsis officinalis,* which in turn showed strong activity against flu, herpes and pox viruses whereas cold water and hot water extracts >180 °F (82 °C) from the fruitbody of the same strain showed no activity against these same viruses.

Moreover, the inventor focuses on using mycelium, not fruitbodies, from traditionally used medicinal macrofungi in the discovery of powerful antiviral properties and structures. This inventor has identified numerous antiviral molecules resident within and expressed extracellularly by the mycelium and extractable with cold water/ethanol, from grain (rice), wood, and lignocellulosic based substrates, using solvents other than hot water to create novel compositions useful for reducing viruses and their cross infectivity to help animal cells from viral invasion and replication.

Optimizing dosage is dependent upon numerous variables. The difference between a medicine and poison is often dosage. Determining the proper dose for antiviral effects will only require routine experimentation because the concentrations of extracts can be simply diluted or concentrated by adjusting ethanol and/or water content. In general, with regard to *Ganoderma lucidum* var. *resinaceum* ("*G.r.*") blends, compositions consisting of 5-95% *G.r.* are preferred, 10-75% is more preferred and 20-50% is most preferred.

The term "effective amount" refers to an amount sufficient to have antiviral activity and/or enhance a host defense mechanism as more fully described below. This amount may vary to some degree depending on the mode of administration, but will be in the same general range. The exact effective amount necessary could vary from subject to subject, depending on the species, preventative treatment or condition being treated, the mode of administration, etc. The appropriate effective amount may be determined by one of ordinary skill in the art using only routine experimentation or prior knowledge in the art in view of the present disclosure.

The average American adult has increased body mass compared to the past, hence a dosage regimen must account for larger individuals than has been historically practiced. For instance, the average American woman weighed 142 (64.4 kg) lbs in 1990; today, the average woman weighs 160 lbs (72.6 kg). Similarly, the average weight for American men in 1990 was 180 lbs, (81.6 kg) and today men average around 195 lbs (88.4 kg). By way of an example, a typical dosage regimen for adults of varying body mass, could be calculated at follows, allowing for considerable flexibility of dosages depending on circumstances known to the science of pharmaceutical dosing.

Typical therapeutic amounts of mycelium grown on rice for a 180 lbs (81.6 kg.) adult, (extracts of individual fungal species and/or combinations of species) are preferably 0.1-20 g/day, more preferably 0.25-10 g/day, and most preferably 0.5-5 g/day.

Typical therapeutic amounts of extracts (individual fungal species and/or combinations of species) preferably deliver 0.1-20 mg extracted materials per kg of body weight, more preferably 0.25-10 mg/kg of body weight and most preferably 0.5-5 mg/kg of body weight.

Typical daily dose therapeutic amounts of active molecules, active compounds or active principal ingredients, including ethyl 7-chloro-2-oxo-4-phenyl-2H-chromen-3-carboxylate, vanillic acid, hispolon, quercetin hydrate, rutin hydrate, syringic acid, trans-cinnamic acid, trans-ferulic acid and conjugate and ionic salts, esters or congeners of vanillic acid, syringic acid, trans-cinnamic acid and *trans-*ferulic acid, for prevention of viral infection preferably range from about 100-200 mg daily (about 1.2-2.4 mg/kg of body weight for a 180 lbs or 81.6 kg adult), preferably divided into two dosage units administered twice per day. A preferred daily therapeutic dose is about 200 mg. The active ingredients ethyl 7-chloro-2-oxo-4-phenyl-2H-chromen-3-carboxylate, vanillic acid, hispolon, quercetin hydrate, rutin hydrate, syringic acid, trans-cinnamic acid, trans-ferulic acid and conjugate and ionic salts, esters or congeners of vanillic acid, syringic acid, trans-cinnamic acid and trans-ferulic acid may be combined with each other in any ratios. In general, compounds with FDA approval are preferred both singly and in blends in any ratio while compounds lacking such approval (such as ethyl 7-chloro-2-oxo-4-phenyl-2H-chromen-3-carboxylate) are less preferred. Typical therapeutic amounts of active molecules, active compounds or active principle ingredients for treating, ameliorating, mitigating, alleviating, reducing and curing a pathogenic virus infection preferably range from about 0.001 to 2.0 g/day, more preferably range from about 0.1 to 1.5 g/day and most preferably range from about 0.25 to 1.4 g/day, preferably divided into two dosage units administered twice per day for a period of time ranging from 10 to 60 days. A preferred daily therapeutic dose for treating a pathogenic viral infection is about 1,000 mg subject to adjustments for weight as above. Typical therapeutic amounts of active ingredients preferably deliver about 0.012 mg to 24.5 mg active ingredient per kg of body weight based on an average body weight of 180 lbs (81.6 kg), more preferably about 1.2 mg to 18.4 mg/kg of body weight and most preferably about 3.1 to 17.2 mg/kg of body weight. A preferred daily therapeutic dose for the potential use of psilocin would be about 0.1 mg per kg of body weight.

**Table 1 Body Weight, Daily Dose and Number of Capsules**

| **Body Weight kg (lbs)** | **Daily Dose** | **Number of Capsules** |
|---|---|---|
| <66 | 800 mg/day | 2 x 200-mg capsules A.M. |
| (<144) | | 2 x 200 mg capsules P.M. |
| 66-80 | 1000 mg/day | 2 x 200-mg capsules A.M. |
| (145-177) | | 3 x 200-mg capsules P.M. |
| 81-105 | 1200 mg/day | 3 x 200-mg capsules A.M. |
| (178-231) | | 3 x 200-mg capsules P.M. |
| >105 | 1400 mg/day | 3 x 200-mg capsules A.M. |
| (231) | | 4 x 200-mg capsules P.M. |

Note that effective ranges/dosages are not expected to be precisely the same for all compounds. Dosages may be optimized with each compound when the pharmacokinetics are studied to see how each compound is metabolized in the gastrointestinal tracts and in the liver through the cytochrome P450 pathways, which may alter the dose ranges. However, these compounds were compared side-by-side to well-studied antiviral drugs within the same dosage ranges recognized by pharmaceutical science, so these dosage levels are predictive and rational.

Delivery systems of these compositions containing one or a plurality of antiviral molecules include, but are not limited to: sprays, capsules, tablets, elixirs, emulsions, lozenges, suspensions, syrups, pills, lotions, epidermal patches, suppositories, inhalers, and injectables, or by other means known to the art of drug delivery. For measured, long term dosing and to achieve a more consistent effect, delayed release delivery systems known to the pharmaceutical industry can be employed. Additionally, these compounds can be combined with other drugs or enzyme suppressants to allow passage through the liver's complex cytochrome P450 and related pathways to yield an effective amount into the blood stream.

The antiviral extracts, active constituents, mycelium and/or other derivatives may be incorporated into foods to produce foods with antiviral properties, useful for protecting animals including humans, dogs, cats, horses, cows, pigs, birds, fish, insects, including bees, and other wild and domesticated animals, from infection.

To the best of this inventor's knowledge, it is unprecedented to have an antiviral extract dually active against viruses that infect humans and viruses that infect honey bees. That the alcohol-water extracts of polypore mushrooms (*Fomes fomentarius, Ganoderma applanatum, Ganoderma resinaceum, Inonotus obliquus, Schizophyllum commune, Trametes versicolor* and *Fomitopsis* species) are active against disparate families of viruses afflicting humans and bees is a strange, unexpected and nonobvious result to those skilled in the art of viral medicines fighting either human or bee viruses. Such novel results are the subject of pending patent applications filed by the inventor, i.e. U.S. Patent Application No. 14/641,432: "Integrative fungal solutions for protecting bees," filed March 8, 2015, and U.S. Patent Application No. 14/247,207: "Integrative fungal solutions for protecting bees and overcoming Colony Collapse Disorder (CCD): methods and compositions," filed April 7, 2014.

An antiviral agent active against one virus does not mean, of course, that there will be antiviral activity against another. Indeed, compounds that have antiviral activity against all viruses are typically poisons, as viruses have specificity responses due to their unique modes of activity, infection, RNA and DNA compositions, transcriptomes and receptor fields on their membranes. Current thinking is there are more species of viruses on earth than all the species of fungi, plants and prokaryotes combined! And a universal antiviral is likely to kill off many beneficial viruses - a nascent subfield in virology. Hence, it is not obvious nor a medically justifiable theory that a hit against one virus means the same compound will be against all the species in the virome.

As viruses mutate and develop tolerances to antiviral medicines, the search for, discovery, and identification of new antiviral molecules is a priority for mitigating pandemics, whether natural or human-made. To this end, the inventor and his team have discovered that aqueous ethanolic extracts of the mycelia of polypore mushrooms native to the forests of the Pacific Northwest demonstrate remarkable activity in the standard assays for evaluating anti-flu, anti-herpes and anti-smallpox drugs. Particularly strong activity was noted against influenza viruses A and B.

Following the Project BioShield Act of 2004, Fungi Perfecti, LLC submitted over 200 hot water fruitbody and aqueous ethanolic extracts of the *in vitro* grown mycelia of mushroom species and strains within to the BioShield BioDefense Program administered by the U.S. Army Medical Research Institute of Infectious Diseases (USAMRIID) and the National Institutes of Health (NIH). Submitted samples were screened for antiviral activity coordinated through the Southern Research Institute (SRI). From the more than 200 samples the inventor provided to BioShield, 2,392 antiviral assays were performed on behalf of the inventor; 1,042 of which were influenza tests. From these 1,042 tests, 13 samples had Sl₅₀>100, about a 1.2% success rate against any virus. Within a species such as Agarikon (*Fomitopsis officinalis*), an extract from one strain from this species, often showed significant antiviral activity against one flu virus, for instance, but not another. In essence, the probability of success proved highly unlikely and the negative results would be discouraging to other researchers bioprospecting large libraries of organisms for novel antivirals.

The protocols for determining antiviral activity are fully described by the National Institutes of Allergy and Infectious Diseases (NIAID) by Greenstone et al., NIAID resources for developing new therapies for severe viral infections, Antiviral Res., Volume 78, Issue 1, April 2008, Pages 51-59. The influenza bioassays were conducted according to Sidwell, RW and Smee, DF, In vitro and in vivo assay systems for study of influenza virus inhibitors, Antiviral Res., Oct. 2000, 48(1): 1-16., hereby incorporated by reference in its entirety.

Advances in determining useful antiviral medicines are increasingly relying upon human cell well assays as some viruses infect humans differently than in animals. While many viruses jump from animals to humans, their etiology is often different, and the results from animal tests are not necessarily translatable, often failing when put into contact with human cells. Hence many animal models are not representative of the potential utility of new antiviral drugs needed for treating humans. NIH Virology has designed a suite of tests which confirms antiviral activity using human cells, and once activity is noted, pharmacokinetical models are used to predict how they will be metabolized within humans. As in the case of vanillic acid, whose safety, has been well established, the most effective route is to go from human cell well assays directly into human studies in the treatment of the shingles (Herpes zoster) virus. Moreover, the growing trends, due to societal norms, is to steer away from animal testing models as much as possible.

Strong activity against orthopox viruses, influenza A, and influenza B was detected in *Fomitopsis officinalis* aka Agarikon, *Ganoderma resinaceum* (=*Ganoderma lucidum* var. *resinaceum*)*,* aka Red Reishi, and *Inonotus obliquus,* aka Chaga, species of mushrooms which contain varying amounts of many of the active principle ingredients described herein, and moreover those specifically cited in the Claims.

As illustrated in Table 2, a percent solution of *Fomitopsis officinalis* extracts as low as 1 - 2 % inhibited virus-induced cellular damage by 50 % (EC₅₀) in the standard assays for screening antivirals. When the crude extract (100 % solution) was diluted by a factor of 10⁶ (0.0001 % solution), a 50 % reduction in cellular damage by pathologically relevant influenza (influenza A and influenza B) and herpes viral (HSV) strains was observed. Selectivity Index (SI) numbers greater than 5 are moderately active, greater than 10 are highly active and greater than 100 are extraordinarily highly active.

**Table 2: Antiviral Activity of EtOH/cold H₂O extracts of mycelia of various compositions of mushroom species submitted to NIH contract labs under the "Bioshield" project. Influenza A and B (Flu A, B) assays were tested in MDCK cells against a Ribavirin control. Herpes Simplex (HSV) assays were tested in HFF cells against am Aciclovir control. Hepatitis C (HCV) assays were tested in Huh7 ET cells against an IFN alpha-2b control. The Selectivity Index (SI) scale is the ratio of cytotoxicity of the agent (CC aka IC) to the selective antiviral activity (EC) such that a SI>10 indicates extraordinarily strong activity.**

| **Flu A - Active Samples with SI > 100** | | | | | | | | | | | | |
|---|---|---|---|---|---|---|---|---|---|---|---|---|
| **Cmpd Name** | **Virus** | | **Strain** | **Assay** | **EC50** | **EC90** | **IC50** | **SI** | **Cntrl EC50** | **Cntrl EC90** | **Cntrl IC50** | **Cntrl SI** |
| HDT3 1x | Flu A (H5N1) | | Vietnam/1203/2004H | Visual | <0.0001 | | >0.1 | **>1000** | 10 | | >320 | >32 |
| Fo-6 25x EtOH only 24 hours | Flu A (H3N2) | | Wisconsin/67/2005 | Neutral Red | <0.0001 | | 0.026 | **>260** | 2 | | >320 | >160 |
| | Flu A (H3N2) | | Wisconsin/67/2005 | Visual | <0.0001 | | 0.032 | **>320** | 1.9 | | >320 | >170 |
| Io-1 25x 33 days | Flu A (H5N1) | | Vietnam/1203/2004H | Neutral Red | <0.0002 | | 0.04 | **>200** | 12 | | >320 | >27 |
| | Flu A (H5N1) | | Vietnam/1203/2004H | Visual | <0.001 | | 0.03 | **>300** | 10 | | >320 | >32 |
| Host Defense | Flu A (H5N1) | | Vietnam/1203/2004H | Neutral Red | <0.0001 | | 0.02 | **>200** | 12 | | >320 | >27 |
| | Flu A (H5N1) | | Vietnam/1203/2004H | Visual | <0.0001 | | 0.03 | **>300** | 10 | | >320 | >32 |
| Gr 25x | Flu A (H5N1) | | Vietnam/1203/2004H | Visual | 0.0002 | | 0.038 | **190** | 3.2 | | >320 | >100 |
| Io-1 25x | Flu A (H5N1) | | Vietnam/1203/2004H | Visual | 0.0005 | | 0.057 | **110** | 3.2 | | >320 | >100 |

| **Flu B - Active Samples with SI > 100** | | | | | | | | | | | | |
|---|---|---|---|---|---|---|---|---|---|---|---|---|
| **Cmpd Name** | | **Virus** | **Strain** | **Assay** | **EC50** | **EC90** | **IC50** | **SI** | **Cntrl EC50** | **Cntrl EC90** | **Cntrl IC50** | **Cntrl SI** |
| Pb-1 25x | | Flu B | Shanghai/361/02 | Neutral Red | <0.0001 | | 0.059 | **>590** | 7.1 | | >320 | >45 |
| Gr 25x EtOH only 24 hrs | | Flu B | Shanghai/361/02 | Neutral Red | <0.0001 | | 0.04 | **>400** | 7.1 | | >320 | >45 |
| | | Flu B | Shanghai/361/02 | Virus Yield | | 1E-04 | | **522** | | 37.72 | | >8.4 |
| Io-1 25x EtOH only 24 hrs | | Flu B | Shanghai/361/02 | Neutral Red | <0.0001 | | 0.035 | **>350** | 7.1 | | >320 | >45 |
| | | Flu B | Shanghai/361/02 | Virus Yield | | 1E-04 | | **223** | | 37.72 | | >8.4 |
| Fo-13 25x EtOH only 24 hrs | | Flu B | Shanghai/361/02 | Visual | 0.0003 | | 0.047 | **150** | 1.7 | | >320 | >190 |
| | | Flu B | Shanghai/361/02 | Virus Yield | | 1E-04 | | **313** | | 37.72 | | >8.4 |
| | | Flu B | Shanghai/361/02 | Visual-CONF | 0.0003 | | 0.047 | **150** | 1.7 | | >320 | >190 |
| G. app 25x EtOH only 24 hrs | | Flu B | Shanghai/361/02 | Visual | 0.0003 | | 0.057 | **180** | 1.7 | | >320 | >190 |
| | | Flu B | Shanghai/361/02 | Visual-CONF | 0.0003 | | 0.057 | **180** | 1.7 | | >320 | >190 |
| Fo-10 25x EtOH only 24 hrs | | Flu B | Shanghai/361/02 | Virus Yield | | 0.0001 | | **171** | | 37.72 | | >8.4 |
| Gr 25x | Flu B | Shanghai/361/02 | | Neutral Red | 0.0004 | | 0.053 | **140** | 7.1 | | >320 | >45 |

| **HSV 1 - Active Samples with SI > 100** | | | | | | | | | | | | |
|---|---|---|---|---|---|---|---|---|---|---|---|---|
| **Cmpd Name** | **Virus** | | **Assay** | | **EC50** | **EC90** | **CC50** | **SI** | **Cntrl EC50** | | | |
| Fo-1 25x EtOH only 3 weeks | HSV-1 | | CPE | | 0.02 | >1 | 3.9 | **195** | 0.3 | | | |

| **HCV** - **Active Samples with SI > 10** | | | | | | | | | | | | |
|---|---|---|---|---|---|---|---|---|---|---|---|---|
| **Cmpd Name** | **Virus** | | **Assay** | | **EC50** | **EC90** | **IC50** | **SI50** | **Cntrl EC50** | **Cntrl EC90** | **Cntrl IC50** | **Cntrl SI50** |
| Ttv EtOH only 24 hrs | HCV | | HCV RNA replicon Confirmatory (Dose Response) | | 5.59 | >10 0 | >100 | **>17.9** | | | | |
| Positive Control IFN alpha-2b | HCV | | HCV RNA replicon Confirmatory (Dose Response) | | | | | | **0.07** | **0.38** | **>2** | **>28.6** |
| Positive Control IFN alpha-2b | HCV | | HCV RNA replicon Confirmatory (Dose Response) | | | | | | **0.12** | **0.54** | **>2.0** | **>16.7** |

| **Key:** | | | | | | | | | | | | |
|---|---|---|---|---|---|---|---|---|---|---|---|---|
| Fo-1 = *Fomitopsis officinalis,* strain #1 | | | | G. app = *Ganoderma applanatum* | | | Io-1 = *Inonotus obliquus,* strain#1 | | | | | |
| Fo-6 = *Fomitopsis officinalis,* strain #6 | | | | Gr = *Ganoderma resinaceum* HDT3 = A blend of Fo-1, Gr, Pb | | | and Pb-1 = *Piptoporus betulinus, strain #1* | | | | | |
| Fo-10 = *Fomitopsis officinalis,* strain #10 | | | | | | | Tv = *Trametes versicolor* | | | | | |
| Fo-13 = *Fomitopsis officinalis,* strain #13 | | | | | | | | | | | | |
| Host Defense = a 16 species blend, containing: *Fomitopsis officinalis, Ganoderma resinaceum, Piptoporus betulinus, Inonotus obliquus, Grifola frondosa, Cordyceps sinensis, Polyporus umbellatus, Agaricus brasiliensis, Phellinus linteus, Schizophyllum commune, Trametes versicolor, Hericium erinaceus, Ganoderma applanatum, Ganoderma oregonense, Fomes fomentarius, Lentinula edodes* | | | | | | | | | | | | |

Fortunately, in 2014, the inventor's application to submit samples for continued antiviral testing was accepted by NIH Virology. However, NIH Virology refused to test extracts from mycelium due to their complexity but would, upon prior approval to submission, accept pure molecules, i.e. "structures" for testing. Pure compounds would only be tested if NIH Virology specialists vetted and approved the new structures as being ones not tested before by NIH, nor having evidence of activity against the viruses selected for study in the scientific literature, in order to eliminate redundancy. Since there can be more than 200,000 compounds resident in mycelia and mushrooms, the inventor was burdened with a seemingly impossible task: which compounds of more than 200,000+ molecules would be active against viruses? To conduct the bioguided fractionation tests standard in pharmaceutical discovery of new drugs typically would take years, as well as enormous laboratory resources.

The inventor, knowledgeable in the decomposition of wood by polypore mushrooms, and knowing that these polypore mushrooms contained polyphenols, focused on 20+ compounds related to polyphenols and related to acids produced in the de-lignification of wood, more specifically coumaric acids and their analogs and related congeners. NIH approved the first submission of 10 of 20 structures the inventor initially proposed for testing. Against tremendous odds, the first tests resulted in 3 of 10 structures being more active against a variety of viruses than the side-by-side positive drug controls used by NIH Virology testing laboratories. Upon the second submission, 5 of the 10 structures showed higher activity against viruses than cidofovir, the positive drug control, in tests against the human papilloma virus (HPV). In perspective, there are no good antivirals against HPV currently on the market, according to personal communication between the inventor and NIH Virology. Although an anti HPV vaccine has been developed, many will not receive it for a wide variety of medical and societal factors. Furthermore, millions of people are already infected with HPV who will not substantially benefit from vaccination (the recommended age for the vaccine is 11-12 years old, and not recommended after the age of 26). Having a novel antiviral that is active against HPV significantly serves public health by reducing the pathogen payload in the ambient infected population, reducing downstream communicability of disease, and in effect reducing deaths from cervical, anal, oral and other cancers.

Compounds were screened by NIH Virology contracted laboratories against a panel of viruses, resulting in significantly high activity against the human papilloma virus (HPV), varicella-zoster virus, norovirus (Norwalk), Epstein-Barr virus and polioviruses. To measure antiviral activity, the SI₅₀ was compared to a control drug for the same virus. The SI₅₀ is simply the CC₅₀, aka IC₅₀ (compound concentration that reduces cell viability by 50%) divided by the EC₅₀ (compound concentration that reduces viral replication by 50%). One sample (hispolon) showed modest activity against Ebola, a single-stranded RNA virus, with a SI₅₀>5.3, promising - as the SI₅₀ of the control drug, favipiravir, has only a SI₅₀>10. In a time of dire need for antivirals against Ebola, constituents related to hispolons merit further study with likelihood of discovering molecules closely related to hispolons that will be more antivirally active than favipiravir's SI₅₀>10.The inventor continues to pursue anti-Ebola compounds isolated from fungi.

A SI₉₀ in the Selectivity Index (SI) scale is the ratio of cytotoxicity of the agent vs. the selective antiviral activity (CC₅₀/EC₉₀) such that a SI₉₀>10 indicates extraordinarily strong activity whereby the compound concentration reduces viral replication by 90% and reduces cell viability by 50%.

Noteworthy is that very few antiviral medicines have significant SI₉₀'s >10. Acyclovir against *Varicella zoster* is a good example to discuss. The SI₉₀ of acyclovir, (SI₉₀=6), is far less active as an antiviral than vanillic acid, claimed by this inventor, which has SI₉₀ >195 against *Varicella zoster* virus, a herpes virus causing "shingles." To achieve such high reduction of virus (90%) greatly underscores that vanillic acid and its analogs (for instance, p-hydroxybenzaldehyde and p-hydroxybenzoic acid) have high potential for becoming useful antivirals, especially since vanillin has a long history of safe use and is widely and safely consumed as a delicious edible flavoring. Vanillin is oxidized, after consumption, into vanillic acid. Vanillic acid or its related analogs can be delivered using nano-encapsulation techniques such as encasing vanillic acid within a biodegradable protein covering so that it passes through the liver into the bloodstream unaltered. Additionally, vanillic acid can be encapsulated with other molecules to allow its passage into the bloodstream post liver metabolism in a therapeutically effective amount. Similarly, any physical or chemical method or means for increasing bioavailability known to the art may be utilized.

Chrysin >98%, *trans*-cinnamic acid >98%, trans-ferulic acid >98%, quercetin hydrate >95%, rutin hydrate >98%, syringic acid >97% and vanillic acid >98% were obtained from TCI Chemicals. Hispolon ≥ 98% was obtained from Enzo Life Sciences. Ethyl 7-chloro-2-oxo-4-phenyl-2H-chromen-3-carboxylate, prepared according to Hwang et al., J. Nat. Prod. 76: 1916-1922 Supporting Information S-28-29 (2013), was obtained from the Department of Medicinal Chemistry and Pharmacognosy, College of Pharmacy, University of Illinois at Chicago, Chicago, Illinois 60612, courtesy of the authors. These compounds exhibited moderate to high antiviral activity according to the NIH Selectivity Index protocol in Table 3:

**Table 3: Active Principal Ingredient (API) molecules with specific antiviral activity compared to the positive antiviral drug controls selected and tested by NIH Virology and their subcontractors.**

| Chemical Name, ARB# | Chemical Structure | Activity |
|---|---|---|
| Ethyl-7-chloro-2-oxo-4-phenyl-2H-chromen-3-carboxylate | 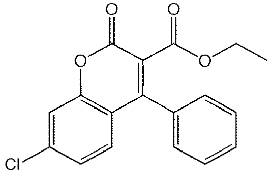 | **Norovirus primary:** |
| | | SI50=25 (Control 2'C- methyl cytidine SI50>16) |
| ARB#14-000863 | | |
| Vanillic acid | 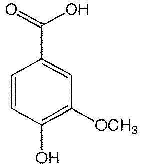 | **Varicella-Zoster primary:** |
| ARB#14-000866 | | SI50>448 (Control acyclovir SI50>652), SI90>195 (Control acyclovir SI90>6) |
| Hispolon | 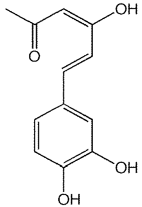 | **Norovirus primary:** |
| ARB#14-000869 | | SI50>13 (Control 2'C-methyl cytidine SI50>16) **Epstein-Barr primary:** SI50=5 (Control cidofovir SI50>10) |
| Chrysin | 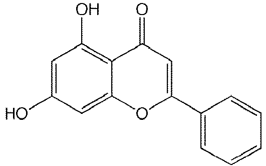 | **Polio primary:** |
| ARB#15-000498 | | SI50>15 (Control pirodavir SI50>22) |
| Quercetin hydrate | 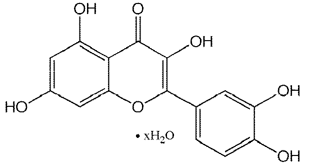 | **Human papillomavirus 11 primary:** SI50>60 (Control cidofovir SI50>12) |
| ARB#15-000501 | | |
| Rutin hydrate | 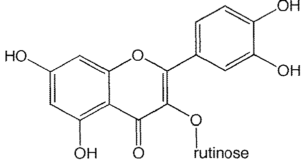 | **Human papillomavirus 11 primary:** SI50>109 (Control cidofovir SI50=12), SI90=5 (Control cidofovir SI90=1) |
| ARB#15-000502 | | |
| Syringic acid | 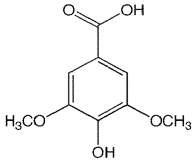 | **Human papillomavirus 11 primary:** SI50>30 (Control cidofovir SI50>12) |
| ARB#15-000503 | | |
| *trans-*cinnamic acid | 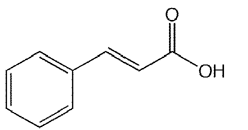 | **Human papillomavirus 11 primary:** SI50>125(Control cidofovir SI50>12) |
| ARB#15-00050 | | |
| *trans* -ferulic acid | 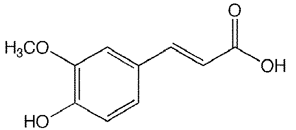 | **Human papillomavirus 11 primary:** SI50>125 (Control cidofovir SI50>12) |
| ARB#15-000505 | | |

Katayama *et al.* (2013) discloses that an enzymatically converted form of vanillic acid is antiviral.¹³ This prior art by Katayama et al. (2013) does not make this inventor's discovery of vanillic acid being active against a herpes virus (*Varicella zoster*) obvious for the following three reasons:

Noroviruses (tested by Katayama) are not related to herpes viruses (tested by the inventor). Norovirus (Norwalk virus) is in the Calciviridae and is a single stranded RNA virus and herpes viruses (*Varicella zoster,* Epstein-Barr, etc.) are in Herpesviridae and are double stranded DNA viruses. These are about as distant as viruses can be. It is highly unlikely that an antiviral molecule would be active against such different viruses, and those skilled in the art of antiviral discovery would not assume activity against herpes viruses as an obvious logical extension of antiviral activity against noroviruses.

Vanillic acid in its pure form was not tested by Katayama, but rather novel enzymatically converted vanillic acid analogs synthesized by rutinases (rutinosylation).

This inventor's NIH tests of vanillic acid (Sample 14-00086) against the norovirus revealed a SI₅₀=1 and SI₉₀=1, meaning there is no activity of inhibition of vanillic acid against the norovirus. This provides strong evidence for the inventor's argument (1) above that activity against herpes viruses is not expected, anticipated, or an obvious extension of observed activity against noroviruses.

The inventor has taken on the lifelong task of comprehensively evaluating the antiviral activity of Agarikon (*Fomitopsis officinalis*), Amadou (*Fomes fomentarius*)*,* Artist Conks (*Ganoderma applanatum* also known as *Ganoderma annulare, Ganoderma brownii*), Chaga (*Inonotus obliquus*), Red Reishi (*Ganoderma lucidum* sensu lato, *Ganoderma resinaceum, Ganoderma sinense, Ganoderma tsugae, Ganoderma oregonense),* the Split Gill Polypore (*Schizophyllum commune*), Turkey Tail (*Trametes versicolor*) and other polypores in the same clades and non-polypore mushrooms in the Agaricales. In pursuit of this goal, the inventor has surprisingly discovered that many of these same extracts of Agarikon (*Fomitopsis officinalis*), Chaga (*Inonotus obliquus*) and Red Reishi (*Ganoderma resinaceum, Ganoderma lucidum*), that reduce viruses afflicting human cells also significantly reduce viruses afflicting other animals, even honey bees (see U.S. Patent Application No. 14/641,432: "Integrative fungal solutions for protecting bees," 2015.). As such, this inventor believes these fungi are deep reservoirs of many novel antiviral molecules, offering a broad bioshield of defense against pathogenic viruses afflicting animals. Derivative of this discovery, the inventor predicts more antiviral molecules will be discovered, many of which are related to the herein described molecules listed in this patent application. The inventor predicts some of these molecules may also be active against viruses afflicting plants and hence useful for protecting agriculture.

These antiviral components are produced by many polypore fungi (polyporales) such as but not limited to *Fomitopsis officinalis, Fomitopsis pinicola, Fomes fomentarius, Inonotus obliquus, Ganoderma applanatum, Ganoderma oregonense, Ganoderma resinaceum, Ganoderma sinense, Ganoderma tsugae, Irpex lacteus, Schizophyllum commune, Trametes versicolor* and gilled fungi (Agaricales) such as *Pleurotus ostreatus, Pleurotus pulmonarius, Pleurotus populinus, Stropharia rugoso-annulata, Stropharia semigloboides, Stropharia ambigua, Psilocybe allenii, Psilocybe azurescens, Psilocybe coprophila, Psilocybe cubensis, Psilocybe cyanescens, Clitocybe odora,* and *Lactarius fragilis.*

The inventor predicts more antiviral molecules will be found within the extracellular and intracellular metabolites related not only to polyphenols and fatty acids, but also with molecules, which in association with each other, increase antiviral activity. Examples include but are not limited to: amylase, amyloglucosidase, betulinic acid, caffeic acid, protocatechuic acid, trans-cinnamic acid, ferulic acid, gallic acid, ellagic acid, lanosterol, inotodiol, trametenolic acids, hispolons (hispidins), hispidin, hypholomine B, inoscavin A, davallialactone, phelligridin D, ergosterols, chrysin, cordycepin, trans-o-coumaric acid, *trans-p-*coumaric acid, ellagic acid dihydrate, ergosterol, linoleic acids, transferulic acid, gallic acid hydrate, hexanal, hispolon, 4-hydroxybenzoic acid, *p-*hydroxybenzaldehyde, 4-hydroxybenzaldehyde, p-hydroxybenzoic acid, quercetin hydrate, rutin hydrate, (including related flavonoid glycosides), shikimic acid, syringic acid, vanillic acid, vanillin, ethyl vanillin, isobutyl vanillin, metabolic precursors of vanillin and vanillic acid, metabolic products of vanillin and vanillic acid, acetovanillone, guaiacol, eugenol, sulphurenic acid, dehydrosulphurenic acid, eburicoic acid, trans-cinnamic acid, trans-ferulic acid, 6-chloro-4-phenyl-2H-chromen-2-one, ethyl 6-chloro-2-oxo-4-phenyl-2H-chromen-3-carboxylate, 7-chloro-4-phenyl-2H-chromen-2-one, ethyl 7-chloro-2-oxo-4-phenyl-2H-chromen-3-carboxylate (and other coumarins), psilocybin, psilocin and their conjugate and ionic pharmaceutical salts, esters, congeners (related chemical substances related to each other by origin, structure, or function), isomers, structural and functional analogs and significantly similar substituted analogs including hydroxylated, acetylated, methoxylated, ethoxylated and halogenated compounds known to those of skill in the art which may prove useful in the practice of this invention, including activity against viruses and oncoviruses. This would include, by way of example, but not of limitation: influenza viruses (H1N1, H3N2, H5N1, H5N2, H5N3, H5N8, H5N9, H7N1, H7N2, H7N3, H7N4, H7N5, H7N6, H7N7, H7N8, and H7N9, H9N1, H9N2, H9N3, H9N4, H9N5, H9N6, H9N7, H9N8, and H9N9), *Herpesviridae* viruses, herpes simplex virus-1, herpes simplex virus-2, human cytomegalovirus, murine cytomegalovirus, *Varicella zoster* virus, Epstein-Barr virus, human herpes virus-6, human herpes virus-8, respiratory and other viruses including SARS coronavirus, respiratory syncytial virus, Ebola virus, Nipah virus, measles virus, adenovirus-5 virus, norovirus, rabies virus, *Arenaviridae,* Tacaribe virus, Pichinde virus, poliovirus, Junin virus, Lassa fever virus, *Bunyaviridae,* Rift Valley fever virus, Punta Toro virus, La Crosse virus, Maporal virus, *Flaviviridae,* dengue virus, West Nile virus, yellow fever virus, Japanese encephalitis virus, Powassen virus, *Togaviridae,* Venezuelan equine encephalitis virus, eastern equine encephalitis virus, western equine encephalitis virus, chikungunya virus, *Picornaviridae,* poliovirus, enterovirus-71, enterovirus-68, coxsackievirus B3, *Papovaviridae,* BK virus, JC virus, papillomavirus, *Poxviridae,* vaccinia virus, cowpox virus, monkeypox virus, polyoma, and hepatic viruses, hepatitis A virus, hepatitis C virus and hepatitis B virus. The compounds are also anticipated to be useful with all animals, including humans, mammals, birds, bats, pigs and bees, and with viral diseases carried by ticks, fleas, flies, mosquitos and other insects and arthropods.

With any new drug, the expense of production and availability are two factors determining whether or not the drug can be commercialized. The active principle ingredients (API) discovered by the author can be purchased "off the shelf" from a variety of chemical supply companies. The following prices are for small test quantities. Buying these *en masse* would drastically reduce the costs associated with each API. Given a target dose of approximately 100-200 mg. per day, the costs of many of these new antiviral molecules, with the exception of hispolon, are extraordinarily inexpensive. If buying masses of these API to fuel a supply chain and meet market demands, given the economies of scale going from 100 mg. to >1000 kg. per purchase order, the costs for each should be reduced at least 10x, and probably more. The following prices are retail prices available for research purposes buying 100 mg. (1/10^{th} of a gram) before negotiation for discounts (USP or pharmaceutical grades may be higher).
Chrysin: TCI Chemicals, >98%, $0.39/100mg
trans-Cinnamic acid: TCI Chemicals, >98%, $0.038/100mg
trans-Ferulic acid: TCI Chemicals, >98%, $0.15/100mg
Hispolon: Enzo Life Sciences, ≥98%, $1280.00/100mg
Quercetin hydrate: TCI Chemicals, >95%, $0.15/100mg
Rutin hydrate: TCI Chemicals, >98%, $0.12/100mg
Syringic acid: TCI Chemicals, >97%, $0.16/100mg
Vanillic acid: TCI Chemicals, >98%, $0.12/100mg

Cidofovir, an injectable antiviral against which many of these molecules (APIs) were favorably compared is very expensive and the generic form currently sells for $ 342 to $ 626 for 5 doses in per vial (5ml) containing 75mg/ml (generic). The least expensive comparative antiviral is acyclovir (now off patent) and sells for $ 7.59 for 200 mg. By way of example, the inventor's APIs against Human Papilloma Virus (HPV), showed high antiviral activity using molecules many orders of magnitude less expensive than cidofovir.

While focusing on the delignification and cellulose-decomposing pathways and byproducts from mushroom mycelium of species from the Polyporales and Agaricales, and studying how to save bees from colony collapse disorder, the inventor observed high attractancy of the extracellular droplets exuding from the mycelium mushrooms, initially of a gilled mushroom in the Agaricales (*Stropharia rugoso-annulata*), when a patch of mycelium of this species was planted in his garden. When the mycelium grows on wood or grains, it decomposes lignin-cellulosic, hemi-cellulosic or cellulosic components and expresses polyphenol-like compounds. Focusing on p-coumaric acid, the inventor scoured the scientific literature, which showed that coumaric acid was essential for activating the gene sequences for the cytochrome p450 detoxification pathways in bees. Constellations of molecules were then explored by the inventor for antiviral activity and, surprisingly, many of these acids showed the strong antiviral activity, as noted within this patent application.

Consideration of metabolic pathways supposedly unrelated to antivirals provided inspiration and guidance in selecting many of the currently disclosed compounds for testing. For example, Terrón et al., Structural close-related aromatic compounds have different effects on laccase activity and on Icc gene expression in the ligninolytic fungus Trametes sp. I-62, Fungal Genet. Biol., 41:954-962 (2004) teaches: "Nine phenolic compounds (p -coumaric acid, ferulic acid, guaiacol, syringol, p-methoxyphenol, pyrocatechol, phloroglucinol, 3,5-dihydroxybenzoic acid, and syringaldazine) were tested for their ability to increase laccase production in the ligninolytic basidiomycete Trametes sp. I-62. All these compounds resulted in increases in laccase activity, with the highest levels being detected in the presence of p -coumaric acid (273-fold) and guaiacol (73-fold)." The inventor, while researching what compounds activate the cytochrome P450 detoxification pathways in bees, and how *Trametes versicolor* mycelium uses enzymes for delignifying lignin (in wood) and cellulose (in paper, grains, starch), found the co-occurring molecules particularly surrounding p-coumaric acid to be interesting to test for antiviral activity. Although the inventor's curiosity in his pursuit of helping bees to overcome colony collapse disorder led him to look at coumaric acid, and this spurred him to send to NIH Virology an assortment of molecules related to coumaric acid, it is interesting to note that, to date, coumaric acid has not demonstrated any antiviral activity. The inventor will continue to look at coumaric acids and their congeners as a source of novel antivirals. Nevertheless, this further speaks that the inventor's discovery being unobvious and indeed bizarre.

With the active principal ingredients put forth by the inventor herein, compounds new to science with these multifaceted properties are first described. All compounds and mixtures containing these compounds can be administered using any method known to the art of pharmaceutical drug delivery, including new nanotechnologies and microbiome therapies allowing for controlled release, enhancement and targeting of medicines.

These active principle ingredients (APIs) can be found in both fungi and plants, and can be combined with each other, or with other drugs, or attached to other molecules to increase their pharmacokinetic effectiveness, specificity, and/or longevity of effects in order to confer a medical benefit. Additionally, The APIs can be embedded within carrier molecules of greater complexity, which upon digestion, allow for the APIs to pass into the blood stream in an amount effective to be impart an antiviral and immune supporting benefit.

More novel antiviral molecules are expected to be discovered, derivative of this invention, from Amadou (*Fomes fomentarius*), Agarikon (*Fomitopsis officinalis*), Red Belted Polypore (*Fomitopsis pinicola*), Artist Conk (*Ganoderma applanatum*), Red Reishi (*Ganoderma lucidum* or *Ganoderma resinaceum*), *Ganoderma sinense,* Chaga (*Inonotus obliquus*), Mesima (*Phellinus linteus*), Split Gill Polypore (*Schizophyllum commune*), Turkey Tail (*Trametes versicolor*) and their closely related species within the same taxonomic clade or clades from which these species evolved, using the methods known to the art of pharmaceutical drug discovery. Compounds discovered by the inventor existing in non-mushroom forming fungi and in bacteria and plants may also impart antiviral activity to other viruses not yet tested. Moreover, combinations of these active principle ingredients and their congeners are expected to also reduce viruses such as hepatitis, flu, SARS (severe acute respiratory syndrome), MERS (Middle Eastern respiratory syndrome), and other viruses, some yet not known to science. This discovery offers an umbrella of protection, an armamentarium, a broad bioshield or "mycoshield" of defense against one or a plurality of viruses causing disease.

### REFERENCE EXAMPLE 1

In tests initiated and by cause of the inventor, the following antiviral activity was reported by NIH contracted virologists. These results show antiviral activity of high significance and potential utility for creating new drugs and new compositions of drugs, treatments, prophylactics, adjuvants, nutraceuticals, animal feeds and dietary supplements. By way of example and not of limitation, these compositions may include pure compounds, nearly pure isolates or fractions, extracts of natural products containing the disclosed molecules and synthesized molecules identical to the naturally occurring molecules, herein described and claimed as novel antiviral agents of significance against multiple viruses.

Pure or nearly pure molecules were submitted by the inventor via his solely owned business Fungi Perfecti, LLC, and his employee Dr. Regan Nally, to Dr. Mark Prichard, University of Alabama (UAB) Birmingham, Alabama 35233 to test for anti-human papillomavirus (HPV) activity via NIH Virology, using protocols conforming to standards established by the National Institutes of Health/Virology. Dr. Mark Prichard's team analyzed the inventor's molecules against HPV (human papillomavirus) using state-of-the-art testing methods utilizing quantitative polymerase chain reaction (DNA)/CellTiter-Glo (Toxicity) protocols.

In general, extracts of those species and strains of mushrooms, or purified molecules occurring within them, or synthetic versions of such molecules, or analogs of such molecules, that are active against viruses with a SI₅₀≥5 are preferred, with SI₅₀>10 being more preferred and SI₅₀>30 being most preferred. Any compound having SI₅₀>100 is considered extremely active and extraordinarily selective.

The primary screening of vanillic acid against *Varicella zoster* (the "shingles" or human herpesvirus 3, HHV-3) virus resulted in high activity with an SI₅₀>448 and SI₉₀>195 compared to the positive drug control acyclovir which, in the same, side by side test, had a SI₅₀>26 and a SI₉₀>1.

This compound, vanillic acid, is produced as a by-product in the delignification of wood by *Fomitopsis, Ganoderma, Inonotus* and other fungi, particularly the wood decomposers. Vanillic acid is also produced through metabolic oxidation of vanillin, the common flavoring, upon ingestion. Vanillin can be synthesized or sourced from a wide variety of plants - even from manure! Vanillin is readily available in quantity, making it a uniquely favorable and flavorable prime candidate for further testing and rapid commercialization, as this is a well-known and safe food ingredient. The amount of vanillic acid, the oxidized form of vanillin, and the correlated dosages for an antiviral based on side-by-side comparisons of vanillic acid to acyclovir, falls well within a safe amount for a 70 kg human, i.e. 700 mg vanillin per day or for an 88 kg American male, 880 mg vanillin per day. The average concentration of vanillin in the vanilla flavoring available to consumers ranges from 0.38 to 8.59 mg/ml. Hence, for a person to gain antiviral benefit from drinking vanilla extract would mean ingesting unrealistic volumes of vanilla extract and at a very high expense.

Vanillin and vanillic acid are common molecules in the plant world, notably *Angelic sinensis* or Dong Quai has some of the highest amounts of vanillic acid found in nature. "The majority of industrially produced vanillin is ingested in the form of food and beverages. Minor amounts are applied topically as skin care products, perfumes, etc. The global use of vanillin in food and beverages imply that almost every human globally is exposed to minute amounts of vanillin by ingestion, although individual doses and exposure can vary due to eating habits and preferences. An Acceptable Daily Intake (ADI) of 10 mg/kg has been agreed between FAO/WHO and EU. For a 70 kg person the ADI is 700 mg vanillin which, as an example, corresponds to minimum of 700 g chocolate, or 7,000 g of ice cream. For the risk assessment it is assumed that even persons with a high intake of vanillin containing food and beverages do not have a vanillin intake above the ADI.^{"14}

However, 700-880 mg of vanillin is within the therapeutic dosage window to impart antiviral effects for a 70-88 kg person. For the average American male, buying an effective dosage of 880 mg. of vanilla from a grocery store is an impractical amount for consumers to ingest using the concentrated vanilla flavorings which are commonly available.

More information can be found in the Handbook of Vanilla Science and Technology (2011), edited by Dahna Havkin-Frenkel and Faith Belanger.

Although naturally occurring, the use of vanillic acid and vanillin as antivirals is novel. Note that vanillin has many analogs and closely related compounds, some of which are described herein as being antiviral and more of which are expected to be antiviral.

There is an emergent need for new antivirals active against *Varicella zoster* and other herpes viruses due to emerging drug resistance as these strains mutate and are cofactors in causing inflammation and carcinogenesis. Vanillic acid, and its closely related acids, offer new sources for antiviral medicines that are inexpensive to make. At a time of crisis when few new safe antiviral drugs are coming onto the market, vanillic acid can be easily manufactured, standardized for potency and studied clinically with little expectation of harm compared to most new antivirals. Moreover, vanillic acid has good pharmacokinetics. Vanillic acid from oral ingestion remains in the blood stream post-digestion for more than 11 hours - a pharmacokinetic advantage because this compound will have prolonged cell-viral contact, which strengthens its potential as an advantageous antiviral drug.

Since up to 95% of vanillin is metabolized within the human body into vanillic acid, vanillin and its enzymatically converted analogs, are also predicted to be primary antiviral agents against viruses, particularly against herpes viruses related to *Varicella zoster* viruses. *Varicella zoster* is one of nine currently known herpes viruses infecting humans, at least three of which are thought to be oncoviruses: herpes virus 4 (HHV-4 = Epstein-Barr); herpes 6 (HHV-6); and herpes 8 (HHV-8)). HHV-6 has been detected in lymphomas, leukemia, cervical cancers, Karposi sarcoma and brain tumors. Herpes viruses are also neurotoxic and often are associated, with or without causality, to the neurodegeneration and neuropathic inflammation often associated with the Alzheimer's disease complex. Moreover, vanillic acid and the other compounds, compositions, and derivatives disclosed herein can upregulate cytochrome enzymatic pathways, specifically in genes coding for cytochrome p450, and the p21 and p53 pathways for up-regulation and production of tumor necrosis proteins. Hence having more than one benefit from these aforementioned APIs can synergistically result in compounded benefits useful to medicine. For medicines to have antiviral, antibacterial, immune enhancing, antioxidant, and tumor de-cloaking properties with P21 and P53 gene activation effects, post ingestion, represent a novel anticancer armamentarium of active agents. The more synergistic benefits we have available for our cells to combat disease, the more likely a positive outcome. Rather than finding a single agent against a single virus, presenting an animal with a menu of benefits provides a broader shield of protection.

Any means, methods or process steps known to the art may be utilized to manufacture the extracts or to extract, isolate or purify an active ingredient or to synthesize and purify the active ingredients of the current invention. "Extracts" as used herein includes crude or purified extracts containing multiple compounds and multiple active compounds or either the supernatant or solids or precipitates isolated from a crude extract or an isolated or purified fraction, ingredient, compound or molecule from an extract.

### EXAMPLE 2

The NIH virology report on the inventor's compounds tested against HPV noted that five samples are "highly active," being designated in red lettering by NIH virology for emphasis. Table 3 (listed previously) shows that 5 of the 10 molecules submitted in one set demonstrated significant antiviral HPV activity, with Selectivity Indexes (SI)>10. Other isoforms related to these compounds are expected to confer even greater antiviral activity and complementary benefits, beyond antiviral activity, making them more useful in medicine for treating diseases.

The primary screening of potential antiviral molecules against the human papillomavirus (HPV) were compared against cidofovir, the positive drug control, which showed a SI₅₀>12; SI₉₀=1. The Selectivity Indexes for testing of the inventor's selected molecules against HPV are as follows: quercetin hydrate SI₅₀>60; rutin hydrate SI₅₀≥109; syringic acid SI₅₀>30, trans-cinnamic acid SI₅₀>125 and trans-ferulic acid SI₅₀>125.

In general, those species and strains of mushrooms, or mushroom mycelia or extracts of mushroom mycelia, or purified molecules occurring within them, or analogs of those molecules, are preferred. Those species, strains, mycelia, extracts or molecules that are active against HPV having a SI₅₀≥5 are preferred, with SI₅₀>10 being more preferred and SI₅₀>30 being most preferred. Any compound having SI₅₀>100 is considered extremely active and extraordinarily selective against HPV, with low toxicity to human cells, and thus has high clinical potential for the prevention of HPV cross infection. Of note is that there are very few antiviral drugs active against HPV, and vaccinations are partially effective, yet controversial, with long term consequences still to be determined. All the above five mentioned molecules selected and submitted on behalf of the inventor greatly exceeded cidofovir's anti-HPV activity.

### REFERENCE EXAMPLE 3

Hispolon (ARB# 14-000869) also showed, according to NIH Virology, 'moderately active' antiviral activity against the Epstein-Barr oncovirus with a SI₅₀= 5 compared to the positive drug control cidofovir which has a SI₅₀>10.

### REFERENCE EXAMPLE 4

Poliovirus infections continue to spread around the world. Although smallpox is thought to be successfully eradicated, polio still survives despite widespread vaccinations. Few effective antiviral drugs have been discovered. Virology tests at Utah State University (USU) Institute for Antiviral Research at Logan, Utah, under contract to NIH Virology, screened numerous antiviral candidates submitted by the applicant. One molecule in particular, chrysin, demonstrated moderate to high levels of antiviral activity inhibiting the polio virus with a SI₅₀>15 compared to the positive drug control, pirodavir, which had a SI₅₀=22. Hence, chrysin and its derivatives and analogs may prove to be new antivirals to help reduce the poliovirus.

### REFERENCE EXAMPLE 5

Very few compounds have shown dual activity against viruses and bacteria. Ethyl 7-chloro-2-oxo-4-phenyl-2H-chromen-3-carboxylate (a synthetic analog of the naturally occurring 6-chloro-4-phenyl-2H-chromen-2-one and ethyl 6-chloro-2-oxo-4-phenyl-2H-chromen-3-carboxylate) and hispolon are two such compounds, not only active against the norovirus (ethyl-7-chloro-2-oxo-4-phenyl-2H-chromen-3-carboxylate SI₅₀=25 and hispolon SI₅₀>13) but also against the bacterium *Mycobacterium tuberculosis,* see "Chlorinated coumarins from the polypore mushroom Fomitopsis officinalis and their activity against Mycobacterium tuberculosis," Hwang et al., J. Nat. Prod. 76: 1916-1922 (2013) (co-authored by the present inventor).

Such dual antiviral and antibacterial activity against the tuberculosis causing bacterium, *Mycobacterium tuberculosis,* and the Norwalk virus (norovirus), a virus notoriously problematic on cruise ships, makes these unique medicines useful for protecting people traveling in close quarters from viral and bacterial infections and useful for protecting and benefitting public and environmental health and reducing the impacts of diseases in animals, particularly humans.

The rapid spread of the norovirus and the high infectivity trends with multi-drug resistant tuberculosis are but one example of a complex disease state wherein people's health is threatened by more than one contagion. As such, ethyl 7-chloro-2-oxo-4-phenyl-2H -chromen-3-carboxylate and hispolon, and compositions containing these compounds, can protect passengers, patients, travelers or people wherever they congregate from multiple assaults from viral and bacterial infections. Congeners and analogs of ethyl 7-chloro-2-oxo-4-phenyl-2H - chromen-3-carboxylate and hispolon are expected to show improved efficacy against viral and bacterial infections. Delivery systems include but are not limited to sprays, capsules, tablets, elixirs, emulsions, lozenges, suspensions, syrups, pills, lotions, epidermal patches, suppositories, inhalers and injectables, or by other means known to the art of drug delivery. For measured, long term dosing and to achieve a more consistent effect, delayed release delivery systems can be employed.

In medicine, conventional thinking is that drugs cannot be dually antiviral and antibacterial. Hence, these compounds, and others related to them, are likely to impart a broader shield of protection from infection caused by pathogenic, infectious viruses and bacteria. These compounds may be the first among many showing dual activity against these two disparate categories of infectious agents. Since the average person is blind to knowing whether an infection is from a virus or a bacterium, and since bacterial infections are notoriously common (and often deadly) subsequent to a viral infection, this invention serves an important function in broadly protecting public health - before infection, at the time of infection or post infection. This discovery may lead, derivatively, to a whole new class of medically useful agents, methods, compositions and treatments.

### REFERENCE EXAMPLE 6

Numerous analogs of hispolons, including hispidin, may be useful as antiviral and antibacterial medicines. At least 26 related analogs are known thus far, and the inventor sees these as being prime candidates for new treatments against viruses, oncoviruses and pathogenic bacteria, and as anti-inflammatories. As such, hispolons offer a unique synergy of benefits for protecting animal health via multiple pathways. Some, but not all of these can be found in Balaji et al., Design, Synthesis and In Vitro Cell-based Evaluation of the Anti-cancer Activities of Hispolon Analogs, Bioorganic & Medicinal Chemistry 23: 2148-2158 (2015).

These compounds may directly, or indirectly, positively influence disease outcomes, as they work via separate pathways and activate sets of receptors, which cumulatively and synergistically enhance immunity, overcome toxicity of xenobiotic toxins, have antiviral and antibacterial properties and potentiate benefits from other drug therapies.

### EXAMPLE 7

Although ethanol and water extracts are illustrated within this invention, it will be obvious that the various solvents and extraction methods known to the art may be utilized.

The extracts may optionally be prepared by methods including extraction with water, alcohols, organic solvents and supercritical and subcritical fluids such as CO₂, and from extracts derived from fermentation with bacteria such as but not limited to *Bacillus subtilis.* Extracts may also be prepared via steam distillation of volatile components, similar to the preparation of "essential oils" from flowers and herbs. Suitable alcohols include those containing from 1 to 10 carbon atoms, such as, for example, methanol, ethanol, isopropanol, n-propanol, n-butanol, 2-butanol, 2-methyl-1-propanol (t-butanol), ethylene glycol, glycerol, etc. Suitable organic solvents include unsubstituted organic solvents containing from 1 to 16 carbon atoms such as alkanes containing from 1 to 16 carbon atoms, alkenes containing from 2 to 16 carbon atoms, alkynes containing from 2 to 16 carbon atoms and aromatic compounds containing from 5 to 14 carbon atoms, for example, benzene, cyclohexane, cyclopentane, methylcyclohexane, pentanes, hexanes, heptanes, 2,2,4-trimethylpentane, toluene, xylenes, etc., ketones containing from 3 to 13 carbon atoms such as, for example, acetone, 2-butanone, 3-pentanone, 4-methyl-2-pentanone, etc., ethers containing from 2 to 15 carbon atoms such as t-butyl methyl ether, 1,4-dioxane, diethyl ether, tetrahydrofuran, etc., esters containing from 2 to 18 carbon atoms such as, for example, methyl formate, ethyl acetate, butyl acetate, etc., nitriles containing from 2 to 12 carbon atoms such as, for example acetonitrile, proprionitrile, benzonitrile, etc., amides containing from 1 to 15 carbon atoms such as, for example, formamide, *N,N*-dimethylformamide, *N,N*-dimethylacetamide, etc., amines and nitrogen-containing heterocycles containing from 1 to 10 carbon atoms such as pyrrolidine, 1-methyl-2-pyrrolidinone, pyridine, etc., halogen substituted organic solvents containing from 1 to 14 carbon atoms such as, for example, bromotrichloromethane, carbon tetrachloride, chlorobenzene, chloroform, 1,2-dichloroethane, dichloromethane, 1-chlorobutane, trichloroethylene, tetrachloroethylene, 1,2-dichlorobenzene, 1,2,4-trichlorobenzene, 1,1,2-trichlorotrifluoroethane, etc., alkoxy, aryloxy, cyloalkyl, aryl, alkaryl and aralkyl substituted organic solvents containing from 3 to 13 carbon atoms such as, for example, 2-butoxyethanol, 2-ethoxyethanol, ethylene glycol dimethyl ether, 2-methoxyethanol, 2-methoxyethyl ether, 2-ethoxyethyl ether, etc., acids containing from 1 to 10 carbon atoms such as formic acid, acetic acid, trifluroacetic acid, etc., carbon disulfide, dimethyl sulfoxide (DMSO), nitromethane and combinations thereof. Extracts may also be prepared via sequential extraction with any combination of the above solvents or methods mentioned herein. Any means, methods or process steps or any solvents known to the art may be utilized to manufacture the extracts of the current invention. The extracts may be further refined by means known to the art to a more potent antiviral form or an active pharmaceutical ingredient.

Preferred drying methods include freeze drying, air drying, infrared drying, spray drying, vacuum drying, membrane drying, sonification drying, vibrational drying, drum drying, light drying and Refractance Window® drying methods and apparati for drying mycelium, extracellular metabolites, natural product extracts and derivatives as disclosed in U.S. Patent No. 4,631,837 to Magoon (1986). Extracts are preferably extracted from living or frozen mycelium and may be cell-free (filtered and/or centrifuged) or not.

### EXAMPLE 8

*Fomes fomentarius, Fomitopsis officinalis, Fomitopsis pinicola, Ganoderma resinaceum, Inonotus obliquus, Piptoporus betulinus, Trametes versicolor, Schizophyllum commune* and other mushroom species disclosed herein or known to the art are cultured and the mycelium grown on rice, barley, flaxseeds or other grains, agricultural debris, or forest products such as sawdust or wood chips (for a list of substrates, See Stamets, Growing Gourmet and Medicinal Mushrooms, 1993, Ten Speed Press, Berkeley, Ca. and Stamets & Chilton, The Mushroom Cultivator, Agarikon Press, Olympia, WA). When the mycelium reaches a mass of growth (preferably after 5 to 60 days growth in fermentation or in solid state fermentation subsequent to inoculation, but well before fruitbody formation) mycelial mass can be extracted through simple aqueous, water/ethanol (both of which are preferred) or ethanol washing of the substrate, or from compression of the substrate, all of which will result in a liquid fluid or capture-able extract including extracellular exudates. These extracts can be utilized as they are, or alcohol (25-50% by volume) may be added to aqueous extracts as a preservative and solvent (which will precipitate water-soluble polysaccharides). The hydroethanolic extract can be evaporated or removed, or the alcohol and water may be evaporated and removed separately. The crude extract can be cell free filtered using a .12-.20 µm filter. This extract can be frozen or dried for future use. Alternatively, non-aqueous or non-ethanolic solvent extracts such as those listed in Example 1 or other solvents known to the art may be utilized, or subcritical or supercritical fluid extracts utilizing, for example, carbon dioxide or water and optional co-solvents such as alcohols, may be utilized, or microwave-assisted extracts may be utilized. Any means, methods or process steps known to the art for inoculating or cultivating a pure mycelial culture on a solid or cellulosic substrate, extracting a mycelial culture, filtering an extract or partially or fully removing the solvent may be utilized.

In one exemplary embodiment for extracting a mushroom mycelium, equal volumes of cold 95% (190 proof) ethyl alcohol and mycelium are mixed and the mixture allowed to soak for a minimum of 15 minutes, preferably several hours, enabling extraction of both ethanol-soluble and water-soluble compounds. Water is then added to adjust the liquid to 30-40% alcohol by volume and the mycelium is separated from the water/ethanol solvent. In general, water-soluble polysaccharides begin to precipitate out of the liquid or supernatant at 22-23% alcohol by volume, with 22-50% alcohol being preferred in the end product, with 25-35% being most preferred. If collecting the precipitate for use, 35-45% EtOH is preferred in order to more completely precipitate solids. Grain alcohol is preferred for preparation of Kosher extracts.

### EXAMPLE 9

The medicinal mushroom mycelium is grown utilizing liquid culture techniques. Whereas growing on rice might have 30-40% conversion of rice to mycelium, liquid vat culture may have essentially complete conversion with >3x more mycelium per unit mass. Hence the liquid vat culture of mycelium and its extracellular metabolites will be easier to utilize in the development of this invention as the process of using vat culture eliminates the need to remove non-metabolized substrate ingredients. Any means, methods or process steps known to the art for inoculating or cultivating a pure mycelial culture on a liquid substrate, extracting a mycelial culture, filtering an extract or partially or fully removing a solvent may be utilized.

### EXAMPLE 10

To make the mycelial extract, use equal volumes of mycelium grown on grain (barley, flaxseed, rice, oats, millet, wheat, rye, corn), seeds, including nuts, sawdust or wood chips (Douglas fir, pines, oaks, birches, cottonwoods, olives) and immerse into a 50:50 water-ethanol solution. Allow to sit at room temperature for two weeks, and then press to expel the liquid extract. Over several days, a precipitate will fall out of the hydroethanolic solution. The hydroethanolic supernatant is drawn off above the pasty precipitate. After several more weeks, or by using a centrifuge, the precipitate further concentrates into a semisolid state. These wet semisolids are removed and heated to 50° C for 6-8 hours while stirring. The wet volume of semisolids is reduced to about 40% of the original wet semisolids. The drying down of the semi-solids into the caramel "honey-like" substance yields about 16% of the original wet solids wet. Therefore, using 1000mL of wet solids (which was 40% of the initial extract) yields about 170mL of thick syrupy caramel like substance. Continued heating and stirring concentrates this substance with noticeably sweeter properties. The extract can be crystallized, powdered, and used as amendment to other treatments. The liquid, semisolid and crystallized forms are noticeably sweet in taste and could be considered a medicinal candy-like substance useful to both animals and people in a wide number of applications. Any means, methods or process steps known to the art for inoculating or cultivating a pure mycelial culture on grains, nuts or woods, for extracting a mycelial culture with a solvent, for precipitating a precipitate or for concentrating a supernatant may be utilized.

### EXAMPLE 11

A mycelial extract is made by extracting fruitbodies or mycelium of basidiomycetous fungi including *Ganoderma resinaceum* in hot water (80-100° C) for several hours and combined with the room temperature (10-30° C) water extraction of *Fomes fomentarius, Fomitopsis officinalis, Fomitopsis pinicola, Ganoderma resinaceum, Inonotus obliquus, Piptoporus betulinus, Trametes versicolor* and/or *Schizophyllum commune* mycelium grown on grain or wood. To these water extracts, ethanol is added to make the solution greater than 22% EtOH (ethanol), preferably 35-45% EtOH. Upon addition of ethanol, polysaccharides precipitate out of solution and settle at the bottom of the extraction vessel. Upon drawing off the supernatant, the precipitated polysaccharides, rich in glycosides, glycoproteins and other 'nectar-like' nutrients, are collected and heated between 50-70° C over several hours, resulting in the creation of a sweet and beneficial residue. Alternately, the supernatant can be stored over several days, which further yields useful precipitating polysaccharides. These precipitates contain complex sugars, antivirals, antibacterials, cytochrome p450 up-regulating coumaric acids and coumarins, and can be combined with other ingredients. Any means, methods or process steps known to the art for extracting a mycelial culture with water, for adding alcohol, for precipitating solids or for separating a supernatant from solids or a precipitate may be utilized.

### EXAMPLE 12

A mycelial extract made from extracting fruitbodies or mycelium of basidiomycetous fungi including *Ganoderma resinaceum* is first soaked in 100% ethanol (1:1 ratio by mass) for 1-7 days. 95% or 190 proof alcohol may optionally be utilized. Upon draining off the ethanol, the mushroom- or mycelial-marc is immersed into hot water (80-100 C) for several hours and combined with the room temperature (10-30 C) water immersion and extraction of *Fomes fomentarius, Fomitopsis officinalis, Fomitopsis pinicola, Ganoderma resinaceum, Inonotus obliquus, Piptoporus betulinus, Trametes versicolor* and/or *Schizophyllum commune* mycelium grown on grain or wood. To these water extracts, the ethanol extracts previously described are added to make the total combined solution greater than 22% EtOH, preferably 35-45% EtOH. Upon addition of ethanol fraction, polysaccharides precipitate out of solution and settle at the bottom of the extraction vessel. P-coumaric acid, being more soluble in ethanol than water, is richer in the ethanolic extracted supernatant. (The ethanolic supernatant, with concentrated p-coumaric acids, is a reservoir of beneficial p450 coding compounds.) This hydroethanolic supernatant can be stored over several days, which further yields a mixture of polysaccharides but which is proportionately higher in p-coumaric acids than the hot water fractions alone. The precipitate also holds p-coumaric acids, and additionally other nutrients, which can be used as described herein. These p-coumaric enriched precipitates also contain complex sugars, antivirals, antibacterials, and families of coumarins, and can be combined with other ingredients in any delivery system desired. Any means, methods or process steps known to the art for extracting a mycelial culture sequentially with alcohol and hot, cold or room-temperature water, for combining alcohol extracts and water extracts or for precipitating solids or for separating a supernatant from solids or a precipitate may be utilized.

### REFERENCE EXAMPLE 13

At the end of May 2015, the inventor supplied 50 kg of freeze-dried mycelium of Agarikon (*Fomitopsis officinalis*) to a free-range chicken farm of 20,000 chickens, in the center of the bird flu pandemic that ravaged Iowa and Minnesota (this was an experimental test provided by the inventor at no charge). The avian influenza strain H5N8 contributed to H5N2 variants to create a new strain of highly communicable and lethal avian influenza. The chickens were fed at a rate of 0.25 gram per day per chicken of Agarikon mycelium - freeze dried and heat sterilized on a rice substrate, made according to the specifications listed in this and the aforementioned patent applications by the inventor, and was applied with their normal feed at a rate of 1.9 lbs/ton.

The chickens not treated, in the surrounding area, tested positive for avian influenza and had to be subsequently euthanized. The chickens fed with Agarikon mycelium survived with no trace of bird flu. An island of immunity in the midst of a pandemic was observed with the Agarikon-treated chickens. The owners of the chicken farm, who are major stakeholders in the chicken industry, were sufficiently impressed to engage the inventor for supplying chickens with Agarikon mycelium to prophylactically treat chickens prior to the next bird flu outbreak. Agarikon mycelium contains many of the antiviral compounds described herein. Containing a complexity of antiviral molecules, the inventor sees Agarikon and the other fungi, especially polypores listed herein, offering a broad bioshield of protection from pathogenic viruses by offering an extract or specialized dried form of mycelium as a feed supplement. The potential market for protecting the chicken and turkey industry is in the hundreds of millions of dollars. Given these initial tests, approximately 1,000 kg of mycelium (a metric ton) is equivalent to 40,000,000 chicken-days of protection (after three weeks of non-detection of the flu virus, the chickens are considered 'bird virus free' and are allowed to be sold for consumption). The Agarikon mycelium can be marketed and sold as a nutraceutical or as a pharmaceutical food standardized to identifiable markers for batch-to-batch consistency. Mycelium, crude or purified extracts of mycelium or isolated active fractions or ingredients may be similarly utilized.

### REFERENCE EXAMPLE 14

A 58-year old male was diagnosed with an unusual (cytokeratin 7 positive) Merkel cell carcinoma (MCC). After various procedures and completion of radiation therapy, a positron emission tomography (PET) scan showed no evidence of disease. During a regular follow-up six months after completion of radiation therapy, a PET scan revealed a liver lesion, confirmed by magnetic resonance imaging (MRI). Histological features were consistent with his primary MCC tumor. Surgery and radiation were not possible given the location of the tumor; the patient refused chemotherapy because of the relatively poor outcomes and significant side effects associated with chemotherapy treatment of MCC. The patient began taking dietary supplements, liver and colon cleanser and Stamets 7®, a blend of medicinal mushroom mycelium [Royal Sun Blazei (*Agaricus brasiliensis* f. *blazei*), Cordyceps (*Cordyceps sinensis* s.l.), Reishi (*Ganoderma lucidum* s.l.), Maitake (*Grifola frondosa*), Lion's Mane (*Hericium erinaceus*), Chaga (*Inonotus obliquus*) and Mesima (*Phellinus linteus*)] and markedly altered his diet by removing meat, eggs and dairy and substituting organic brown rice, beans, sautéed vegetables and freshly prepared juices of organic vegetables. Five weeks after beginning these alternative approaches, a MRI revealed complete remission of his liver metastasis, and he has remained asymptomatic for a total of 53 months, with the most recent scan showing no evidence of disease. See Vandeven et al., Complete Spontaneous Regress of Merkel Cell Carcinoma Metastatic to the Liver: Did Lifestyle Modifications and Dietary Supplements Play a Role?, Glob. Adv. Health Med., 1(5): 20-21 (2012). Antiviral components within the Stamets 7® formulation may be responsible for the reduction of MCC and may have helped this patient recover from the ensuing cancer.

### REFERENCE EXAMPLE 15

A complex mixture of mycelium of 17 medicinal mushroom species in a product called MyCommunity® and a 7 species blend called Stamets 7® under the Host Defense® brand of Fungi Perfecti (P.O. BOX 7634, Olympia, WA 98507) has been attributed by several customers to alleviate Lyme disease symptoms. Within this mixture, Lion's Mane, *Hericium erinaceus,* and *Cordyceps subsessilis,* have been anecdotally reported by Lyme disease victims to result in relief of symptoms. Lyme disease is caused by *Borrelia* species, particularly the *Borrelia burgdorferi* bacterium, and cohorts, which can co-infect, causing inflammation and lowering immunity, making patients more susceptible to latent or opportunistic infections and carcinogenesis. The species and compounds mentioned herein, therefore, hold promise in new methods and compositions for drug and nutraceutical uses for treating patients suffering from Lyme disease and the related pathologies, symptomologies, and co-infections seen with people with this debilitating, chronic illness. Antibacterial components within the Stamets 7® and MyCommunity® formulations may be responsible for the reduction of Lyme disease and may help relieve symptoms.

### REFERENCE EXAMPLE 16

A physician reported that his hepatitis C viral counts became undetectable after taking a two month regimen of Agarikon Host Defense® capsules containing *Fomitopsis officinalis* mycelium grown on rice (500 mg per capsule). Reprinted, with permission is an exact copy of his case report.

"Here are the facts of my case.

I was diagnosed with hepatitis C in 2000 or 2001. An abnormal liver panel had alerted the MD to check for that virus, as that's about the time that medicine was realizing its spread.

How I got the virus I've never figured out, but I had worked on an inpatient psychiatric unit in SoCal in 1987/88. After that I managed large outpatient mental health clinics down there where a significant number of our patients had this virus. Another possibility is that I got it from a major surgery I had in 1992. In any case, the MD in 2001 said that since my viral load was low, I should wait until better treatments were available before seeking care.

As you probably know, excellent treatments recently became available (though expensive). My internist agreed this January to check my viral load again and although it was still relatively low, he agreed to refer me to a gastroenterologist anyway. That fellow did a liver panel and then at my pleading referred me on to the pharmacist for the course of medication. That's how Kaiser Permanente® does it - a specially-trained pharmacist manages a treatment program. It starts with a baseline check of the viral load, then the daily medication. As soon as the viral load drops to zero, the medications are stopped.

The week before I was to attend the class, the pharmacist ordered another HCV lab test to establish the baseline. On the day of the class, I was literally in the facility, walking to the classroom when the pharmacist called my cell phone. She said the lab test results had just come in and somehow, for some reason, it showed no virus present. She was stumped for an explanation, but said that I couldn't start the medication because I had no viral load to try to reduce.

I called the gastro back next day and said maybe there had been a lab error or something. It had been so hard to get the referral I didn't want to lose this chance to get rid of the virus. He agreed to have the test redone right away, just in case. A week later he called to say that sure enough, the first test was correct. There was no viral load - I'm apparently virus free. He seemed uncertain but said he's heard stories of patients whose immune system was able to clear the virus somehow, but he had never heard of anyone who had carried a viral load for more than 10 years and then have it just disappear like this. He said he had reviewed my records and the viral load had been consistent between first identification and the most recent test in January 2015. But it was gone now, seven months later. And the load was still zero on re-test two weeks later. He said I should be happy.

And it is indeed a relief, as you can imagine. I thought hard for an explanation. It dawned on me that during the spring of this year, I'd taken the Agarikon capsules for maybe two months. Other than that, I was taking curcumin and a vitamin, but had been taking those for years. The only change was adding the mushroom capsule.

I'd heard an interview with you on the radio. (I don't recall what show, but it was in the middle of the night when I couldn't sleep). You didn't say anything about HCV but you had mentioned the antiviral effect of certain mushrooms. I did a literature search and came across an article or patent of yours (I forget which) in which HCV was specifically mentioned, which is why I ordered the Agarikon from your company.

So that's my story. Obviously it's not a lab-controlled experiment, but I've thought hard about other possible explanations and don't have many. I hadn't changed any other lifestyle or dietary habit. So either this remission is due to (a) meditation and prayer, (b) random good fortune, (c) the Agarikon, or (d) some combination of those.

I used two 60-capsule bottles of Agarikon between April and June. I always took one capsule in the morning before breakfast, and sometimes a second capsule in the evening before sleep, but that wasn't consistent. We were down in SoCal in June and July and I wasn't taking the capsules there, but the lab tests that came back negative were done in August (done at the Kaiser facility in Fontana, California). So any beneficial effect must have been in the spring

Feel free to share my story as appropriate, but I'd prefer to stay anonymous of course."

### EXAMPLE 17

Any of the antiviral mycelium, extracts or molecules or their analogs described within this invention can be combined with CBD (cannabidiol) by any means or methods known to the art to provide a dual, synergistic benefit for reducing oncoviruses and up-regulation of immune system pathways, resulting in the cumulative benefit of reducing viral burdens and reducing carcinogenesis. Moreover, the natural products such as mushrooms, fungal mycelium and extracts of fungal mycelium containing the antiviral molecules described herein can be combined with CBD in its purified forms, or with other cannabinoids, or with its natural forms, such as with *Cannabis* species, or extracts thereof, for medical benefit. Additionally, foods can be designed with natural substances containing these aforementioned compositions and antiviral pharmaceutical agents, with and without CBD, specifically to appeal to consumers for maintaining health and preventing and curing diseases. There are at least 85 cannabinoids currently known, any one or combination of which can be utilized for creating a composition of ingredients for medical benefit. Moreover, these ingredients can be combined without making medical claims and conforming to the Dietary Supplement Health and Education Act (DSHEA) of 1994, such as but not limited to supportive statements such as: in support of immunity, in support of innate health states in healthy individuals, in support of a healthy microbiome, and in support of healthy genetic expression. Hispolons, polyphenols, mycoflavonoids, and the antiviral acids shown herein are prime candidates for combining with CBDs to create novel component mixtures. The rationale for combining CBDs and fungal immune enhancing and antiviral constituents is that both CBDs and fungal polysaccharides act to potentiate the immune system. Their mechanisms of action are complementary; respective receptor sites are able to cascade reactions that are similar to and that are different from one another, allowing the interplay of these agonists to tune the immune system according to one's state of health.

CBD from *Cannabis* significantly complements the immune modulating capacities of fungal polysaccharides. Fungal polysaccharides (fungal PS) are agonists for a few key pattern recognition receptors such as TLRs 2, 4, 6 and Dectin-1. These receptors are expressed on immune cells that regulate cell-mediated immunity, such as macrophages, NK cells, and others. Activation of these cells initiates cross-talk with the complement system and, current research suggests, humoral branches of the immune system. Agonist activity at these receptor sites activates MAPK and MyD88 pathways, activating Nf-kappaB. Fungal PS also activate Th1 cells, which coordinate the cell mediated immune response.

CBD is a weak agonist (with low affinity) at the CB2 receptor site, which is expressed on all immune cells and tissues (i.e. tonsils, spleen). The pharmacology of CBD is still being investigated. From what is currently known, CBD downregulates TNF-alpha. This is of interest, as TNF-alpha can be upregulated by fungal PS. CBD is also a weak agonist at GPR55, also known in some circles as the CB3 receptor. This receptor is expressed on a diverse array of cells in the body, and is increasingly being researched for its role in endocannabinoid homeostasis (appetite, memory and mood) and oncogenesis.

Perhaps the most intriguing interaction between CBD and fungal PS lies with T-lymphocytes. Fungal PS activate the Th1 arm of lymphocyte activity. CBD suppresses certain aspects of lymphocyte activity. CBD primarily induces apoptosis by activating the ER-mediated ROS pathway in primary lymphocytes. This yields a net anti-inflammatory effect and is considered to be the mechanism of the anti-arthritic effects of the compound. The full scope of CBD's effects on immune function is still being characterized, and appears to be context-dependent (specifically, receptor density and target cell population).

CBD has additional pharmacological characteristics, including activity at TRPV1 (involved in nociception) and 5-HT1A. The latter suggests a natural pairing with neurologically active and seratonergic mushrooms species like *Hericium erinaceus* or *Ganoderma lucidum.* CBD has been researched for neuroprotective activities, and is a known antipsychotic, anxiolytic and antidepressant. CBD has also been studied for a very wide range of anticancer actions, including induction of apoptosis (via activation of capsase 3, 8 and 9), antiproliferative activity, anti-angiogenesis and prevention of tumor migration and invasion. At high doses (1g/day), CBD has demonstrated antineoplastic effects *in vitro.*

CBD appears to round out and complement the effects of mushroom based ingredient on immune function.

### References/Key Reading

Cabral GA, Rogers TJ and Lichtman AH. Turning Over a New Leaf: Cannabinoid and Endocannabinoid Modulation of Immune Function. Journal of Neuroimmune Pharmacology, 10(2):193-203 (2015).
Hassan S, Eldeeb K, Millns PJ, Bennett AJ, Alexander SPH and Kendall DA. Cannabidiol enhances microglial phagocytosis via transient receptor potential (TRP) channel activation. British Journal of Pharmacology, 171(9): 2426-2439 (2014).
Kaplan BLF, Springs AEB and Kaminski NE. The Profile of Immune Modulation by Cannabidiol (CBD) Involves Deregulation of Nuclear Factor of Activated T Cells (NFAT). Biochemical pharmacology, 76(6): 726-737 (2008).

### EXAMPLE 18

Captive honey bees (*Apis mellifera*) were presented with sugar water (typically 50% sugar (sucrose or corn syrup) and 50% water), to which a percentage, based on mass, of mycelial extracts were added at varying concentrations. The net total overall viral pathogen particle counts of bees receiving mycelium extracts in their sugar water at 0.1% and 1%, showed a dose-dependent reduction in overall bee viruses after one week of treatment. The ethanol-water extracts were made using the methods previously described in U.S. Patent No. 8,765,138 and U.S. Patent Application No. 14/641,432. Viral counts were conducted using assays described in U.S. Patent Application No. 14/641,432: "Integrative fungal solutions for protecting bees" filed March 8, 2015. The viruses screened included chronic paralysis virus (CPV), acute bee paralysis virus (ABPV), Israeli acute paralysis virus (IAPV), Kashmir bee virus (KBV), black queen cell virus (BQCV), cloudy wing virus (CWV), sacbrood virus (SBV), deformed wing virus (DWV), Kakugo virus, invertebrate iridescent virus type 6 (IIV-6), Lake Sinai viruses (LSV1 and LSV2) and tobacco ringspot virus (TRSV). The ethanol-water extracts from the mycelia of the following species showed a net decrease of virus by 9% for *Trametes versicolor* at 10% concentration, 56% for *Fomitopsis pinicola* at 1% concentration, 68% for *Fomes fomentarius* at 1% concentration, 72% for *Inonotus obliquus* at 1% concentration, and 87% for *Ganoderma lucidum* (= *Ganoderma lucidum* var. *resinaceum*) at 0.1% concentration whilst the Rice Control Extract showed a 63% increase in the viral load at 1% concentration in the same week. Replicated trials were run side-by-side at the same time in the same room for accurate comparisons. The inventor suggests that the antiviral molecules described in this patent may be useful for lessening pathogenic viruses in bees and are within the scope of these inventions. Moreover, since the tobacco ringspot virus is a plant virus that also harms bees, the antivirals described herein may be useful for combating viruses that harm plants.

### EXAMPLE 19

*Fomes fomentarius* mycelium grown on birch sawdust and extracted in cold water and ethanol (35% ETOH), then evaporated in front of a HEPA filter at 22 C, for 48 hours, reduced volumes by 90%. Other known means and methods for evaporating, concentrating or removing a solvent may be similarly utilized. This evaporated concentrate was then presented to bees via their feed water. At a .01% concentration by mass, the Deformed Wing Virus (DWV) was reduced, on average by ∼1000:1 with one sample demonstrating a 1,000,000+ to 1 reduction. (Research results provided under subcontract by bee experts Dr. Jay Evans, USDA, in collaboration with Dr. Steve Sheppard, Washington State University (WSU), both of whom have remarked that they had never seen such antiviral activity in the many compounds they know or have tested against bee viruses. (Personal Communications.) This remarkable reduction in viral infection from DWV suggests room temperature or cold water & ethanol extracts of wood grown mycelium is a more potent antiviral than extracts derived from mycelium grown on rice. However, that the rice grown mycelium of several polypore species listed in this patent application also significantly reduces viruses in bees is strong evidence that the core pathway for the expression of antiviral compounds is not exclusively dependent on wood (high in lignin) but is enhanced by endogenous compounds within wood.

Unexpectedly, the extracts from the mycelium grown on birch sawdust showed selectivity against the Deformed Wing Virus (DWV) but much less activity against the Lake Sinai Virus (LSV), underscoring the importance of cross species specificity factors for making the most useful extracts.

Moreover, some but significantly less activity of the birch control extracts against bee viruses was observed but is explainable as, upon PCR DNA analysis, two polypore species, already described and predicted to have antiviral activity in pending patents, i.e. *Trametes versicolor* and a Stereum species, *Chondrostereum purpureum,* was found to be naturally resident in the birch sawdust control used for the alcohol-water control, but interestingly, although the DNA primers for *Fomes fomentarius* was used, *Fomes fomentarius* DNA not detected. The inventor notes that the natural variability of populations of fungi within birch wood sawdust makes natural sawdust extracts unsuitable as an antiviral control compared to the purposeful cultivation of selected polypore species on sterilized wood which can then be subsequently extracted from sawdust or other biodegradable materials, utilizing methods for compositions described within the scope of this patent.

### EXAMPLE 20

Lactobacillus, Acidophilus and Bifidobacteria species can be combined with the mycelium, APIs, or with extracts of the mycelium containing the APIs listed herein, to increase efficacy of the antiviral components, increase bioavailability, facilitate absorption and catalyze forms to increase activity and benefits to hosts challenged with viral pathogens. Moreover, *Trametes versicolor* (Turkey Tail) and *Ganoderma lucidum* (Reishi) are prebiotics favoring beneficial bacteria in the microbiome. As such, these and other beneficial bacteria can be grown with or upon *Trametes versicolor and Ganoderma* species mycelium. Hence these combinations can be used to help facilitate bacterial activation and complex quorum sensing that can improve the efficacy of the APIs listed herein, improving benefits to the virus host organism. Moreover, the polypore species and their mycelial extracts enhance the growth of beneficial Pichia yeast in the gastrointestinal system while suppressing pathogenic yeasts such as *Candida albicans.* Hence extracts from these selected polypore species such as but not limited to the genera Fomitopsis, Ganoderma, Inonotus, Trametes can be prebiotics for both beneficial bacteria and beneficial yeasts improving the health of patients' microbiomes. Any other means or methods for increasing efficacy or increasing bioavailability or facilitating absorption known to the art may additionally be utilized.

### EXAMPLE 21

Any of the active principal ingredients or compositions containing these aforementioned APIs that would be diminished through oxidization can be taken with monoamine oxidase (MAO) inhibitors to help maintain antiviral efficacy. Using oxidase inhibitors will allow better survival of the original APIs through the cytochrome P450 pathways especially via the liver. Numerous natural sources of MAO's can be utilized in combinations with the APIs, with the extracts of mycelium containing these APIs, or with other compositions containing these APIs to increase bioavailability, passage or potency. Plants that can be utilized include but are not limited to *Glycyrrhiza glabra* (licorice root), *Acacia catechu* (catechu plant), *Ginkgo biloba* (ginkgo) Leaf, *Passiflora incarnata* (passionflower) Plant, *Peganum harmala* (Syrian rue) root and seed, *Curcuma longa* (turmeric) root, *Piper methysticum* (kava root), *Hypericum perforatum* (St. John's wort), and *Banisteriopsis caapi* (yage).

### EXAMPLE 22

Various methods can be utilized to increase the production of antivirals from growing mycelium *in vitro,* within enclosed laboratories, reducing new drug discovery and commercialization costs.

As fungi rot wood, breaking down lignin, they also weep water, rich in p-coumaric acid and other nutraceutical compounds. The more p-coumaric acid, the more laccases (enzymes that degrade lignocellulose) are expressed by the mycelium, the more the wood rots, the more fungal polysaccharides (sugars) are produced and ultimately the more these compounds will be in the fungal exudates. Once UV light stimulates the process of signaling the mycelium into primordia formation, laccases decrease and *p*-coumaric acid degrades into *p-*hydroxybenzoic acid, which is closely related to vanillic acid and its metabolic precursors and products.

Interestingly, many of the grains preferred for mycelial spawn production for mushroom industry (see Growing Gourmet & Medicinal Mushrooms by the inventor, Paul Stamets, 1993, 2000, Ten Speed Press, Berkeley) are also rich sources of *p*-coumaric acid and may be useful in antiviral compositions. The primary phenolic acids in rice grain were identified as *p*-coumaric acid, ferulic acid, and sinapinic acid. Hence rice is a good feedstock substrate upon which to grow mycelium to produce the novel antivirals the inventor has discovered.

*p*-Coumaric acid is not only in the grains preferred for mushroom spawn production but is also generated during the normal life cycle of mushrooms, especially prior to primordia formation. *p*-Coumaric acid is a potent inhibitor of tyrosinase, the enzyme essential for melaninization. The presence and abundance of *p*-coumaric acid interferes with the production of darkly colored pigments. Ultraviolet light stimulates the photodecomposition of *p*-coumaric acid, enabling melanization and triggering primordia formation. Once UV light stimulates the process of signaling the mycelium into primordia formation, laccases decrease and *p*-coumaric acid degrade into *p*-hydroxybenzoic acid, which is closely related to vanillic acid, and other antiviral compounds discovered by the inventor such as ethyl vanillin, caffeic acid, protocatechuic acid, *trans*-cinnamic acid, ferulic acid, gallic acid, ellagic acid, lanosterol, inotodiol, trametenolic acids, hispolons (hispidins), hispidin, hypholomine B, inoscavin A, davallialactone, phelligridin D, ergosterols, chrysin, cordycepin, *trans*-coumaric acid, ellagic acid dihydrate, linoleic acids, *trans*-ferulic acid, gallic acid hydrate, hexanal, 4-hydroxybenzoic acid, *p*-hydroxybenzaldehyde, *p*-hydroxybenzoic acid, quercetin hydrate, rutin hydrate, (including related flavonoid glycosides), shikimic acid, syringic acid, acetovanillone, guaiacol, eugenol, sulphurenic acid, dehydrosulphurenic acid, eburicoic acid, stigmasterol and beta-sitosterol, including but not limited to other polyphenols and their analogs that are described herein. It should be noted that the mycelium can be in transitional state prior to, during and post light exposure, so a combination of these active antiviral ingredients can co-occur, or be controlled via light exposure and nutrient limitations.

Light stimulation also triggers the production of psilocybin and psilocin in the mycelium of, for instance *Psilocybe cyanescens, Psilocybe cubensis* and *Psilocybe cyanescens.* The "off/on" production of psilocybin, psilocin, baeocystin, nor-baeocystin and other associated alkaloids from the mycelium caused by light exposure (particularly UV) are interrelated to the production of p-coumaric acid and the resultant metabolic expression of tyrosinase coding for melanin, especially prior to, during and after the time of primordia formation. Hence, this inventor suggests that psilocybin, psilocin, 4-acetoxy dimethyltryptamine, baeocystin, nor-baeocystin and other associated alkaloids may have medicinal properties key to the production or use of novel antivirals not yet discovered by science but discovered or predicted by this inventor. Animals such as humans and bees might benefit from using the mycelium of psilocybin producing mushrooms as a source for novel medicinal agents. The transition of the mycelium upon controlled light exposure, especially blue light in the 300-420 nanometers ranges, affords the development of a customized suite of active ingredients, from which an extract be created or combined with active pharmacological molecules for a net benefit for the patient (human or animal) that consumes this unique combination.

### REFERENCE EXAMPLE 23

Since several viruses described within the scope of this invention cause neuropathy (Epstein Barr, Herpes (Varicella) zoster, herpes viruses, Hepatitis C, polio, West Nile, cytomegalovirus, as way of example and not limitation) and that psilocin and its related analogs cause neurogenesis, the combination of the aforementioned active antiviral molecules discovered by the inventor can be uniquely combined with psilocybin, psilocin, 4-acetoxy dimethyltryptamine, baeocystin, nor-baeocystin to create a unique drug combination that could reduce, prevent, cure or remedy the neuropathic damage from viruses, including peripheral and central nervous system neuropathies. This inventor suggests that these combinations could not only repair neuropathy but improve or maintain intelligence and cognitive health as patients (humans or animals) age. Such a unique combination could prove useful for helping patients suffering from a wide range of neuropathy related illnesses, such as, by way of example and not limitation, Alzheimer's, dementia, bipolar syndrome, Parkinson's and complex neurodiseases encompassed by "Parkinsonisms". Suggested daily to weekly dosages for an average weight adult human male, for instance, would be in the range of:
1 gram of vanillic acid with 30 mg. of psilocybin for a high dose
1/4 gram of vanillic acid with 7.5 mg. of psilocybin for a moderate dose.
1/8 gram of vanillic acid with 3.75 mg. of psilocybin for a low or maintenance dose.

The facultative ratios of using a combination of vanillic acid in combination with psilocybin can be from 1:1 to 100:1 vanillic acid to psilocybin.

Should mushrooms containing 1 % psilocybin be used, the active constituent(s) can be extracted effectively by allowing ice to slowly melt over chopped mushrooms, or using water at or near freezing in an ice slurry and cold water extracted over 2-4 days at 1-2 degrees C. using a proportion of 100 ml. of water to 10-100 grams of mushrooms. The resultant extract, low in confounding beta glucans and other polysaccharides, has translucent deep bluish color. Such 'blue juice' can be freeze-dried, rotary evaporated, or dried by other means not detrimental to psilocybin or psilocin or analog content. Such dried material can be analyzed for standardization to allow drug formulation within prescribed parameters necessary for the FDA or other drug agencies to grant approval for formulation as an approved medicine. Notably, this near freezing cold water extraction method is diametrically contrary to conventional thinking and practices advocated by experts in the mushroom industry. Additionally, a wide range of polar and nonpolar solvents may be additionally employed for greater enhancement of ingredients with anti-neuropathic effects. Preservatives and excipients can be added. The extract can be dried via frost free drying, freeze drying, rotary evaporation, drum drying, ultrasonification, or by any means known to the pharmaceutical industry.

### EXAMPLE 24

As an example, but not one of limitation, the mycelium of *Auricularia auricula* (*A. auricularia-judae*), when grown in culture is whitish and lacks melanin but contains p-coumaric acid. When the mushroom mycelium is exposed to light, the mycelium bio-transforms to create dark brown fruitbodies, which are higher in melanin as they mature, with *p*-coumaric acid, an inhibitor of melanin, concurrently declining. This is one example and is a strong argument for the benefit of using lightly colored to yellowish mycelium, pre-melaninization, and often rich in mycoflavonoids, as a source of mycelium for making antiviral extracts due to its innate p-coumaric acid content compounded by the native content of *p*-coumaric acid in the grains that are used for spawn production for growing mycelium. Interestingly, the ideal interface for capturing the best benefits from mycelium for its nutraceutical and *p*-coumaric acid contents is a short window, often of just a few days in length, before and directly after light exposure, but before dark colored fruitbody development beyond the white primordial stage.

Exposing mycelium grown on rice to blue light (ultraviolet, UV) in the 280-420 nanometer wavelengths, for a short window of time, lasting for a short duration of only 1-5 days, can help create and potentiate the antiviral agents described herein. The intensity of light can range from 50-1,000 lux. By incubating the sterilized rice being actively colonized by the mycelium in plastic bags, which have grown out for a minimum of 1 week and up to 16 weeks, UV light exposure lights can be placed directly above and below horizontally shaped bags for maximum light exposure. The plastic bags can be selected for allowing these blue light wavelengths to reach the mycelium. The mycelium can undergo a phase-change in response to light stimuli into producing derivative antiviral agents that are mentioned in this invention (it is to be expected that during this transitional period, the mycelium may contain varying mixtures of antivirally active compounds). This method and derivative improvements utilizing darkness, light and dark/light cycles as appropriate can potentiate the production of antiviral molecules, some of which are intermediates during the melanization pathways activated by light exposure at specific wavelengths. This opens possibilities for customizing the output of specific antiviral molecules using precise wavelengths, exposure times and intensities of light for manufacturing and potentiating antiviral production from mycelium. Lights can be pulsed and/or sequenced with varying wavelengths for exposing mycelium. The mycelium can also be subsequently agitated to cause new growth spurts, causing differentiation of hyphae with multiple nuclei per cell and hyper-expression of extracellular metabolites containing these antivirals. Moreover, antivirals may be emitted differentially over time, allowing for windows of harvesting by washing the mycelium using cold EtOH and H₂O or other solvents and processes known to the art of natural product extraction. Any method or apparatus for providing appropriate light may be utilized.

### EXAMPLE 25

The production of active principle ingredients against viruses from mycelium can be additionally enhanced by vibrational actions, including but not limited to pulsed sonic vibration - ultrasonification - in combination with API stimulating UV wavelengths. Specific UV spectra and vibrations can be customized for enhancing antiviral yields. Additionally, combinations of fungi, bacteria and plants (algae) may be utilized. Guilds of fungi, bacteria and plants (algae, in particular) can be orchestrated to create a quorum emitting antiviral and antimicrobial compounds useful in medicine. The interplay of these organisms in concert will elicit novel immune modulators and antiviral compounds useful in medicine. As genomic science evolves, quorums of organisms can be designed specifically to better medically benefit the individual from these and related antivirals based upon the genomic 'personality' or constitution of that individual. Any method or apparatus for providing vibrational or sonic actions may be utilized. Preparation of final products can be accomplished using any methods known to the pharmaceutical or food processing industries.

### EXAMPLE 26

Compounds or extracts from mycelium could induce viral replication of bacteriophages in the microbiome that might help support the discovery of selective yet indirect action against pathogenic bacteria and viruses. In addition to mycelium producing small molecules that directly inhibit viral replication, the mycelium may also produce compounds that rally specific viruses against pathogenic bacteria; modulate bacteria (and indirectly viruses) through quorum sensing induction or inhibition; and select for bacteriophages that may result in antibacterial effects without those components actually testing positive for antibacterial activity in the absence of bacteriophages.

### EXAMPLE 27

Subsequent to this inventor's discovery, a suite of much needed, customized dermatological lotions, sprays, dermal patches, ingestibles, or injectables can now be created using combinations of several of the active molecules and novel extracts from mycelium. Dermatological lotions, sprays and dermal patches can be now devised that will useful for repairing nerve damage, reducing inflammation and viral populations while slowing the growth or eradicating tumors by assisting in the induction of apoptosis of tumors or by bolstering native immune response. HPV (Human Papilloma Virus), MCC (Merkel Cell Carcinoma), HHV-8 (Human herpes virus 8 also known as *Kaposi sarcoma-associated herpes virus* (KSHV)) and HIV (Human immunodeficiency virus) can cause an array of skin cancers, including squamous and basal cell skin cancers and melanoma. Some cancers may first present as genital warts. More associations between viruses and skin cancer are likely, and hence these formulas can make use of these unique combinations of ingredients. Alternately, these formulas can be ingested in the form of pills, used as an injectable, nasal or oral sprays, used within suppositories, within carrier nano molecules, or by any effective delivery systems known to the art of medicine.

Exemplary compositions can be produced as shown below. The following proposed formulas (ratios are by mass) utilize a combination of elements of this inventor's novel discoveries. Examples of formulation can include ratios of active ingredients with mycelial extracts in ranges of 1:1:1:1 to 1:10:100:1000, or 1:1:1:1:1 to 1:10:100:1000:10,000.
Lotion for Treating Melanoma Skin Cancer:
   85% Aloe Vera
   5% Vanillic Acid
   4% Glycerin
   2% *Fomitopsis officinalis* extract
   2% *Inonotus obliquus* extract
   2% *Phellinus linteus* extract
   Lotion for Treating Shingles (*Herpes zoster*):
   85% Aloe Vera
   6% Vanillic Acid
   5% *Fomitopsis officinalis* extract
   4% Glycerin
Lotion for Treating HPV associated Skin Cancers & Genital Warts:
   80% Aloe Vera
   4% Glycerin
   4% *Fomitopsis officinalis* extract
   3% Rutin hydrate
   3% Trans-cinnamic acid
   3% Trans-ferulic acid
   3% *Inonotus obliquus* extract.
High Potency Lotion for Treating HPV associated Skin Cancers & Genital Warts:
   70% Aloe Vera
   4% Glycerin
   4% Rutin hydrate
   4% Trans-cinnamic acid
   4% Trans-ferulic acid
   4% quercetin hydrate
   4% syringic acid
   4% *Fomitopsis officinalis* extract
   2% *Inonotus obliquus* extract.
High Potency Ingestible or Injectable or Lotion for Reducing Herpes & HPV viruses
   40% Vanillic Acid
   13% Rutin hydrate
   13% Trans-cinnamic acid
   13% Trans-ferulic acid
   11% *Fomitopsis officinalis* extract
   5% quercetin hydrate
   5% syringic acid
High Potency Broad Spectrum Ingredients for Antiviral Ingestible or Injectable or Lotion for Reducing Herpes, HPV, Flu, Epstein Barr, and Hepatitis viruses
   30% Vanillic Acid
   10% Rutin hydrate
   10% Trans-cinnam ic acid
   10% Trans-ferulic acid
   10% *Fomitopsis officinalis* extract
   10% *Inonotus obliquus extract.*
   10% *Ganoderma lucidum*
   5% Quercetin hydrate
   5% syringic acid

Exemplary embodiments include:
One or more fungal extracts; and one or more pharmaceutically acceptable excipients.
One or more fungal extracts; and one or more solvents.
One or more fungal extracts; and an emollient.
One or more fungal extracts; one or more pharmaceutically acceptable carriers; and one or more solvents.

Exemplary pharmaceutical compositions comprise one or more fungal extracts in an aqueous or ethanol solvent and one or more flavors, colorants, sweeteners, thickeners, or preservatives, wherein the composition is a tincture, elixir, or capsule dosage from.

Additional pharmaceutical excipients useful for the compositions as described herein include, for example, the following: Acidifying agents (acetic acid, glacial acetic acid, citric acid, fumaric acid, hydrochloric acid, diluted hydrochloric acid, malic acid, nitric acid, phosphoric acid, diluted phosphoric acid, sulfuric acid, tartaric acid); Alkalizing agents (ammonia solution, ammonium carbonate, diethanolamine, diisopropanolamine, potassium hydroxide, sodium bicarbonate, sodium borate, sodium carbonate, sodium hydroxide, trolamine); Antifoaming agents (dimethicone, simethicone); Antimicrobial preservatives (benzalkonium chloride, benzalkonium chloride solution, benzethonium chloride, benzoic acid, benzyl alcohol, butylparaben, cetylpyridinium chloride, chlorobutanol, chlorocresol, cresol, dehydroacetic acid, ethylparaben, methylparaben, methylparaben sodium, phenol, phenylethyl alcohol, phenylmercuric acetate, phenylmercuric nitrate, potassium benzoate, potassium sorbate, propylparaben, propylparaben sodium, sodium benzoate, sodium dehydroacetate, sodium propionate, sorbic acid, thimerosal, thymol); Antioxidants (ascorbic acid, ascorbyl palmitate, butylated hydroxyanisole, butylated hydroxytoluene, hypophosphorous acid, monothioglycerol, propyl gallate, sodium formaldehyde sulfoxylate, sodium metabisulfite, sodium thiosulfate, sulfur dioxide, tocopherol, tocopherols excipient); Buffering agents (acetic acid, ammonium carbonate, ammonium phosphate, boric acid, citric acid, lactic acid, phosphoric acid, potassium citrate, potassium metaphosphate, potassium phosphate monobasic, sodium acetate, sodium citrate, sodium lactate solution, dibasic sodium phosphate, monobasic sodium phosphate); Chelating agents (edetate disodium, ethylenediaminetetraacetic acid and salts, edetic acid); Coating agents (sodium carboxymethylcellulose, cellulose acetate, cellulose acetate phthalate, ethylcellulose, gelatin, pharmaceutical glaze, hydroxypropyl cellulose, hydroxypropyl methylcellulose, hydroxypropyl methylcellulose phthalate, methacrylic acid copolymer, methylcellulose, polyvinyl acetate phthalate, shellac, sucrose, titanium dioxide, carnauba wax, microcrystalline wax, zein); Colorants (caramel, red, yellow, black or blends, ferric oxide); Complexing agents (ethylenediaminetetraacetic acid and salts (EDTA), edetic acid, gentisic acid ethanolamide, oxyquinoline sulfate); Desiccants (calcium chloride, calcium sulfate, silicon dioxide); Emulsifying and/or solubilizing agents (acacia, cholesterol, diethanolamine (adjunct), glyceryl monostearate, lanolin alcohols, mono- and di-glycerides, monoethanolamine (adjunct), lecithin, oleic acid (adjunct), oleyl alcohol (stabilizer), poloxamer, polyoxyethylene 50 stearate, polyoxyl 35 castor oil, polyoxyl 40 hydrogenated castor oil, polyoxyl 10 oleyl ether, polyoxyl 20 cetostearyl ether, polyoxyl 40 stearate, polysorbate 20, polysorbate 40, polysorbate 60, polysorbate 80, diacetate, monostearate, sodium lauryl sulfate, sodium stearate, sorbitan monolaurate, sorbitan monooleate, sorbitan monopalmitate, sorbitan monostearate, stearic acid, trolamine, emulsifying wax); Filtering aids (powdered cellulose, purified siliceous earth); Flavors and perfumes (anethole, benzaldehyde, ethyl vanillin, menthol, methyl salicylate, monosodium glutamate, orange flower oil, peppermint, peppermint oil, peppermint spirit, rose oil, stronger rose water, thymol, tolu balsam tincture, vanilla, vanilla tincture, vanillin); Humectants (glycerol, hexylene glycol, , sorbitol); Plasticizers (e.g., castor oil, diacetylated monoglycerides, diethyl phthalate, glycerol, mono- and di-acetylated monoglycerides, propylene glycol, triacetin, triethyl citrate); Polymers (e.g., cellulose acetate, alkyl celluloses, hydroxyalkyl, acrylic polymers and copolymers); Solvents (acetone, alcohol, diluted alcohol, amylene hydrate, benzyl benzoate, butyl alcohol, carbon tetrachloride, chloroform, corn oil, cottonseed oil, ethyl acetate, glycerol, hexylene glycol, isopropyl alcohol, methyl alcohol, methylene chloride, methyl isobutyl ketone, mineral oil, peanut oil, propylene carbonate, sesame oil, water for injection, sterile water for injection, sterile water for irrigation, purified water); Sorbents (powdered cellulose, charcoal, purified siliceous earth); Carbon dioxide sorbents (barium hydroxide lime, soda lime); Stiffening agents (hydrogenated castor oil, cetostearyl alcohol, cetyl alcohol, cetyl esters wax, hard fat, paraffin, polyethylene excipient, stearyl alcohol, emulsifying wax, white wax, yellow wax); Suspending and/or viscosity-increasing agents (acacia, agar, alginic acid, aluminum monostearate, bentonite, purified bentonite, magma bentonite, carbomer, carboxymethylcellulose calcium, carboxymethylcellulose sodium, carboxymethylcellulose sodium 12, carrageenan, microcrystalline and carboxymethylcellulose sodium cellulose, dextrin, gelatin, guar gum, hydroxyethyl cellulose, hydroxypropyl cellulose, hydroxypropyl methylcellulose, magnesium aluminum silicate, methylcellulose, pectin, polyethylene oxide, polyvinyl alcohol, povidone, alginate, silicon dioxide, colloidal silicon dioxide, sodium alginate, tragacanth, xanthan gum); Sweetening agents (aspartame, dextrates, dextrose, excipient dextrose, fructose, mannitol, saccharin, calcium saccharin, sodium saccharin, sorbitol, solution sorbitol, sucrose, compressible sugar, confectioner's sugar, syrup); Surfactants (simethicone); Tablet binders (acacia, alginic acid, sodium carboxymethylcellulose, microcrystalline cellulose, dextrin, ethylcellulose, gelatin, liquid glucose, guar gum, hydroxypropyl methylcellulose, methylcellulose, polyethylene oxide, povidone, pregelatinized starch, syrup); Tablet and/or capsule diluents (calcium carbonate, dibasic calcium phosphate, tribasic calcium phosphate, calcium sulfate, microcrystalline cellulose, powdered cellulose, dextrates, dextrin, dextrose excipient, fructose, kaolin, lactose, mannitol, sorbitol, starch, pregelatinized starch, sucrose, compressible sugar, confectioner's sugar); Tablet disintegrants (alginic acid, microcrystalline cellulose, croscarmellose sodium, crospovidone, polacrilin potassium, sodium starch glycolate, starch, pregelatinized starch); Tablet and/or capsule lubricants (calcium stearate, glyceryl behenate, magnesium stearate, light mineral oil, sodium stearyl fumarate, stearic acid, purified stearic acid, talc, hydrogenated vegetable oil, zinc stearate); Thickening agents (gelatin having a Bloom strength of 50-100); Tonicity agent (dextrose, glycerol, mannitol, potassium chloride, sodium chloride); Vehicle: flavored and/or sweetened (aromatic elixir, compound benzaldehyde elixir, iso-alcoholic elixir, peppermint water, sorbitol solution, syrup, tolu balsam syrup); Vehicle: oleaginous (almond oil, corn oil, cottonseed oil, ethyl oleate, isopropyl myristate, isopropyl palmitate, mineral oil, light mineral oil, myristyl alcohol, octyl dodecanol, olive oil, peanut oil, persic oil, sesame oil, soybean oil, squalane); Vehicle: solid carrier (sugar spheres); Vehicle: sterile (Bacteriostatic water for injection, bacteriostatic sodium chloride injection); Viscosity-increasing (see suspending agent); Water repelling agent (cyclomethicone, dimethicone, simethicone); and/or solubilizing agent (benzalkonium chloride, benzethonium chloride, cetylpyridinium chloride, docusate sodium, nonoxynol 9, nonoxynol 10, octoxynol 9, poloxamer, polyoxyl 35 castor oil, polyoxyl 40, hydrogenated castor oil, polyoxyl 50 stearate, polyoxyl 10 oleyl ether, polyoxyl 20, cetostearyl ether, polyoxyl 40 stearate, polysorbate 20, polysorbate 40, polysorbate 60, polysorbate 80, sodium lauryl sulfate, sorbitan monolaurate, sorbitan monooleate, sorbitan monopalmitate, sorbitan monostearate, tyloxapol). This list is not meant to be exclusive, but instead merely representative of the classes of excipients and the particular excipients that may be used in oral dosage forms as described herein. Any methods known to the art for formulating extracts or active principal ingredients into lotions, soaps, etc. may be utilized.
¹ Proietti FA and Carneiro-Proietti AB, Catalan-Soares BC and Murphy EL, Global epidemiology of HTLV-I infection and associated diseases, Oncogene, 24(39): 6058-68 (2005).
² Aoki, M., T. Motomu, A. Fukushima, T. Hieda, S. Kubo, M. Takabayashi, K. Ono, and Y. Mikami, Antiviral substances with systemic effects produced by Basidiomycetes such as Fomes fomentarius, Bioscience, Biotechnology, and Biochemistry. 57(2): 278-293 (1993).
³ Piraino, F., & C.R. Brandt, Isolation and partial characterization of an antiviral RC-183, from the edible mushroom Rozites caperata, Antiviral Research 43: 67-78 (1999).
⁴ Sarkar, S., J. Koga, R.J. Whitley, & S. Chatterjee, Antiviral effect of the extract of culture medium of Lentinula edodes mycelia on the replication of herpes simplex virus 1, Antiviral Research, 20(4): 293-303 (1993).
⁵ Collins, R.A. and T.B. Ng, Polysaccharopeptide from Coriolus versicolor has potential for use against human immunodeficiency virus type 1 infection, Life Sciences, 60: 383-387 (1997).
⁶ Brandt, C.R. and F. Piraino, Mushroom antivirals, Recent Research Developments for Antimicrobial Agents and Chemotherapy, 4: 11-26 (2000).
⁷ Stamets, P., New anti-viral compounds from mushrooms, HerbalGram. 51: 24-27 (2001).
⁸ Stengler, M., The Health Benefits of Medicinal Mushrooms, p.6, Basic Health Publications, 2005.
⁹ Li et al., Complete mitochondrial genome of the medicinal mushroom Ganoderma lucidum, PLoS ONE 8(8):e72038 (2013).
¹⁰ Seong-Kug Eo, Young-So Kim, Chong-Kil Lee and Seong-Sun Han, Antiviral activities of various water and methanol soluble substances isolated from Ganoderma lucidum, Journal of Ethnopharmacology 68 (1-3): 129-136 (1999).
¹¹ Stamets, P., Novel antimicrobial from mushrooms, HerbalGram 54: 28-32 (2002).
¹² Stamets, *Supra* note 7, at 24, 27.
¹³ Katayama S, Ohno F, Yamauchi Y, Kato M, Makabe H, Nakamura S, Enzymatic synthesis of novel phenol acid rutinosides using rutinase and their antiviral activity in vitro, J. Agric. Food Chem., 61(40): 9617-22 (Epub 2013 Sep 25).
¹⁴ Vanilin, OECD SIDS, UNEP Publications, p.9 (1996).

## Claims

1. A composition for use in treating a pathogenic virus infection in a patient in need thereof comprising one or more of syringic acid, trans-cinnamic acid, trans-ferulic acid, salts thereof, esters thereof, or combinations thereof, wherein the pathogenic virus is human Papillomavirus (HPV).

2. A composition for use according to claim 1, wherein the composition has an antiviral effect selectivity index (SI₅₀= CC₅₀/EC₅₀) against the pathogenic virus SI₅₀≥100.

3. A composition for use according to claim 1 or 2, wherein syringic acid, *trans-*cinnamic acid and/or *trans*-ferulic has an antiviral effect Selectivity Index 50 (SI₅₀) ≥ 10 against the HPV.

4. A composition for use according to any of the preceding claims, wherein the syringic acid has an antiviral effect Selectivity Index 50 (SI₅₀)≥ 30 against the HPV.

5. A composition for use according to any of the preceding claims, wherein *trans*-cinnamic acid and/or *trans*-ferulic acid has an antiviral effect Selectivity Index 50 (SI₅₀)≥ 100 against the HPV.

6. A composition for use according to any of the preceding claims, wherein the composition additionally has an antibacterial effect.

7. A composition for use according to any of the preceding claims to be administered for a period of time from 10 to 60 days to a patient suffering from the pathogenic virus infection at a daily dose of from 0.001 to 2 grams of the composition.

8. A composition for use according to claim 7, wherein the daily dose is from 0.1 to 1.5 grams per day, such as from 0.25 to 1.4 grams per day preferably about 1,000 mg per day.

9. A composition for use according to any one of the preceding claims, for treating, ameliorating, mitigating, alleviating, reducing or curing a virus infection and formulated into a dosage form selected from sprays, capsules, tablets, elixirs, emulsions, lozenges, suspensions, syrups, pills, lotions, epidermal patches, suppositories, inhalers, and injectables.

10. A composition for use according to any one of the preceding claims additionally comprises CBD (cannabidiol).

## Patentansprüche

1. Zusammensetzung zur Verwendung bei einem Behandeln einer pathogenen Virusinfektion bei einem Patienten, der dies benötigt, umfassend Syringasäure, trans-Zimtsäure, trans-Ferulasäure, Salze davon, Ester davon und/oder Kombinationen davon, wobei das pathogene Virus humanes Papillomavirus (HPV) ist.

2. Zusammensetzung zur Verwendung nach Anspruch 1, wobei die Zusammensetzung einen Selektivitätsindex (SI₅₀= CC₅₀/EC₅₀) der antiviralen Wirkung gegen das pathogene Virus SI₅₀≥100 aufweist.

3. Zusammensetzung zur Verwendung nach Anspruch 1 oder 2, wobei Syringasäure, trans-Zimtsäure und/oder *trans-*Ferulasäure einen Selektivitätsindex 50 (SI₅₀)≥10 der antiviralen Wirkung gegen das HPV aufweist.

4. Zusammensetzung zur Verwendung nach einem der vorhergehenden Ansprüche, wobei die Syringasäure einen Selektivitätsindex 50 (SI₅₀)≥30 der antiviralen Wirkung gegen das HPV aufweist.

5. Zusammensetzung zur Verwendung nach einem der vorhergehenden Ansprüche, wobei trans-Zimtsäure und/oder *trans*-Ferulasäure einen Selektivitätsindex 50 (SI₅₀)≥100 der antiviralen Wirkung gegen das HPV aufweist.

6. Zusammensetzung zur Verwendung nach einem der vorhergehenden Ansprüche, wobei die Zusammensetzung zusätzlich eine antibakterielle Wirkung aufweist.

7. Zusammensetzung zur Verwendung nach einem der vorhergehenden Ansprüche, die einem Patienten, der an der pathogenen Virusinfektion leidet, über einen Zeitraum von 10 bis 60 Tagen in einer Tagesdosis von 0,001 bis 2 Gramm der Zusammensetzung verabreicht werden soll.

8. Zusammensetzung zur Verwendung nach Anspruch 7, wobei die Tagesdosis von 0,1 bis 1,5 Gramm pro Tag beträgt, wie etwa von 0,25 bis 1,4 Gramm pro Tag, vorzugsweise etwa 1000 mg pro Tag.

9. Zusammensetzung zur Verwendung nach einem der vorhergehenden Ansprüche zum Behandeln, Verbessern, Mildern, Lindern, Verringern oder Heilen einer Virusinfektion und formuliert in einer Dosierungsform, die aus Sprays, Kapseln, Tabletten, Elixieren, Emulsionen, Lutschtabletten, Suspensionen, Sirupen, Pillen, Lotionen, Epidermalpflastern, Zäpfchen, Inhalatoren und Injektionsmitteln ausgewählt ist.

10. Zusammensetzung zur Verwendung nach einem der vorhergehenden Ansprüche, die CBD (Cannabidiol) zusätzlich umfasst.

## Revendications

1. Composition destinée à être utilisée dans le traitement d'une infection par un virus pathogène chez un patient en ayant besoin, comprenant l'acide syringique et/ou l'acide trans-cinnamique et/ou l'acide trans-férulique et/ou leurs sels et/ou leurs esters et/ou leurs combinaisons, le virus pathogène étant le papillomavirus humain (HPV).

2. Composition destinée à être utilisée selon la revendication 1, dans laquelle la composition a un indice de sélectivité pour l'effet antiviral (SI₅₀= CC₅₀/EC₅₀) contre le virus pathogène SI₅₀≥ 100.

3. Composition destinée à être utilisée selon la revendication 1 ou 2, dans laquelle l'acide syringique, l'acide *trans*-cinnamique et/ou *trans*-férulique a un indice de sélectivité pour l'effet antiviral 50 (SI₅₀) ≥ 10 contre le HPV.

4. Composition destinée à être utilisée selon l'une quelconque des revendications précédentes, dans laquelle l'acide syringique a un indice de sélectivité pour l'effet antiviral 50 (SI₅₀) ≥ 30 contre le HPV.

5. Composition destinée à être utilisée selon l'une quelconque des revendications précédentes, dans laquelle l'acide *trans*-cinnamique et/ou l'acide *trans*-férulique a un indice de sélectivité pour l'effet antiviral 50 (SI_{5O}) ≥ 100 contre le HPV.

6. Composition destinée à être utilisée selon l'une quelconque des revendications précédentes, dans laquelle la composition a en outre un effet antibactérien.

7. Composition destinée à être utilisée selon l'une quelconque des revendications précédentes, à administrer pendant une période de 10 à 60 jours à un patient atteint de l'infection virale pathogène à raison d'une dose quotidienne de 0,001 à 2 grammes de la composition.

8. Composition destinée à être utilisée selon la revendication 7, dans laquelle la dose quotidienne est de 0,1 à 1,5 gramme par jour, comme de 0,25 à 1,4 gramme par jour, de préférence d'environ 1 000 mg par jour.

9. Composition destinée à être utilisée selon l'une quelconque des revendications précédentes, pour traiter, améliorer, atténuer, soulager, réduire ou guérir une infection virale et formulée sous une forme posologique choisie parmi les atomiseurs, capsules, comprimés, élixirs, émulsions, pastilles, suspensions, sirops, pilules, lotions, timbres épidermiques, suppositoires, inhalateurs et injectables.

10. Composition destinée à être utilisée selon l'une quelconque des revendications précédentes, comprenant en outre du CBD (cannabidiol).
